# EUROPEAN PATENT APPLICATION

(11) **EP 4 245 792 A1**
(43) Date of publication of application: **20.09.2023**
(21) Application number: 21891854.8
(22) Date of filing: 09.11.2021
(51) Int. Cl.: C08G 69/02, A61K 31/785, A61K 47/60, A61P 1/16, C08G 65/333

(54) **COPOLYMER CONTAINING POLY(ETHYLENE GLYCOL) AND POLY(L-AMINO ACID DERIVATIVE), MICROPARTICLES THEREOF, AND USE THEREFOR IN PHARMACEUTICAL COMPOSITION**

(30) Priority: 11.11.2020 JP 2020187688
(71) Applicant: University of Tsukuba, Ibaraki 305-8577 (JP)
(72) Inventor: NAGASAKI, Yukio, Tsukuba-shi, Ibaraki 305-8577 (JP); LEE, Yaroslav, Tsukuba-shi, Ibaraki 305-8577 (JP); VONG, Binh Long, Tsukuba-shi, Ibaraki 305-8577 (JP); KODA, Yuta, Tsukuba-shi, Ibaraki 305-8577 (JP); DING, Yuanyuan, Tsukuba-shi, Ibaraki 305-8577 (JP)
(74) Representative: Cabinet Nony
(86) International application number: PCT/JP2021/041160
(87) International publication number: WO 2022/102608

(57) **Abstract**

An object of the present invention is to provide an assembled body of ornithine low in toxicity, effective even in oral administration and high in bioavailability. Another object of the present invention is to provide an ornithine assembled body which is effective in preventing or treating hepatic disorders. Another object is to provide a condensate obtained by introducing an acyl group or the like into an ornithine side chain amino group of polyethylene glycol-b-polyornithine, and to provide a novel assembled body not based on PIC.

## Description

### Technical Field

The present invention relates to a copolymer containing poly(ethylene glycol) and poly(L-amino acid derivative), in particular, a copolymer containing a poly(ornithine) segment or containing a poly(aspartic acid ester), poly(acylated arginine) or poly(ornithine)-co-poly(aspartic acid) segment, microparticles thereof, and use thereof in a pharmaceutical composition.

### Background Art

In patients with hepatic disorders such as hepatic cirrhosis, pathological conditions such as hyperammonemia due to abnormal urea cycle may be developed. It has been reported that L-ornithine, when administered to such patients, not only functions as a substrate for ureagenesis, but also activates urea cycle enzymes including carbamyl phosphate synthetase (CPS) and ornithine transcarbamylase (OTC), resulting in improved urea cycles and reduced ammonia levels (Non-Patent Document 1).

It has been reported that, thus, L-ornithine and a mixture of L-ornithine and L-aspartic acid (LOLA) have a beneficial effect of reducing an ammonia concentration.

On the other hand, in Non-Patent Document 2, it has been confirmed, by a double-blind, randomized, placebo-controlled study, that infusion of the amino acid mixture is extremely weak in efficacy. This is considered to be because water-soluble low-molecular-weight amino acids have a low amount of uptake into the living body and are extremely rapidly metabolized.

In order to solve such problems, the inventors have so far designed a copolymer (polycationic polymer) of poly(ethylene glycol) and poly(arginine), and proposed a polyion complex (PIC) obtained by combining the copolymer with a polyanion such as chondroitin sulfate (Patent Document 1). In addition, the inventors have demonstrated that the retention in blood and accumulation in the liver of a self-assembling drug composed of PIC in which ornithine is incorporated (abbreviated as Nano^{ORN}) are greatly improved, and further that, upon administration of Nano^{ORN} to mice with acute hepatic disorders caused by acetaminophen (APAP), not only was the blood ammonia significantly increased by APAP hepatic disorders reduced to a normal level but levels of AST and ALT, which are hepatic function markers, were significantly reduced, resulting in an improved liver function (Non-Patent Document 3). In addition, it has been confirmed that Nano^{ORN} brings about a liver function improving effect also in NASH model mice.

Thus, by incorporating L-ornithine into a self-assembling drug and subcutaneously administering the drug, the bioavailability of L-ornithine was improved and the liver function improving effect could be obtained.

On the other hand, Nano^{ORN} using PIC as a driving force is unstable against changes in ionic strength and pH, and may be disadvantageous for oral administration, for example, it may liberate a polycation and cause toxicity.

### Citation List

### Patent Literature

Patent Document 1: WO 2016/167333

### Non-Patent Document

Non-Patent Document 1: Nutr. Res. 28 (11) 738 (2008)
Non-Patent Document 2: Gastroenterology 136 (7) 2159 (2009)
Non-Patent Document 3: Poly(ornithine)-based self-assembling drug for recovery of hyperammonemia and damage in acute liver injury, Long B. Vong, Yota Ibayashi, Yaroslav Lee, Dai-Nghiep Ngo, Yuji Nishikawa, Yukio Nagasaki, Journal of Controlled Release, 310, 74-81 (2019).

### Summary of Invention

### Technical Problem

In order to solve the above problems, it is an object of the present invention to provide an assembled body of ornithine low in toxicity, effective even in oral administration and high in bioavailability. Another object of the present invention is to provide an ornithine assembled body which is effective in preventing or treating hepatic disorders.

### Solution to Problem

The inventors have found that, by newly introducing an acyl group or the like into an ornithine side chain amino group of poly(ethylene glycol)-b-poly(ornithine), a copolymer capable of forming a novel assembled body which does not require formation of PIC can be provided. Also, the inventors have found that the thus-provided copolymer is made into a composition with a copolymer containing another poly(ethylene glycol) segment and a poly(side chain protected L-amino acid) segment, and thus the natures, functions or effects of the former copolymer may be enhanced.

Accordingly, the present invention provides the following aspects.
(1) A copolymer represented by Formula (I): 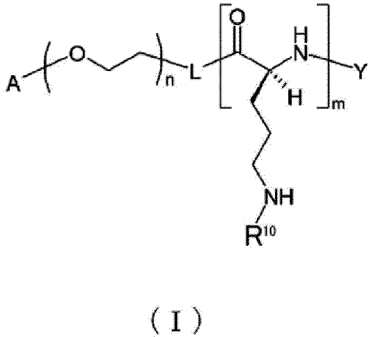 wherein
   A represents:
   (i) a hydrogen atom, an unsubstituted or substituted C₁-C₁₂ alkyl group, an unsubstituted or substituted C₁-C₁₂ alkoxy group, or an unsubstituted or substituted aryl group,
      when the A is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-; or
   (ii) formula 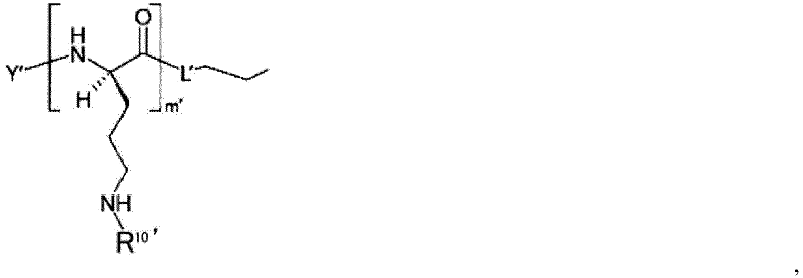 where
      L' independently represents a linking group;
      Y and Y' independently represent a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
      where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
      when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
      R¹⁰ and R¹⁰' are hydrogen atoms or (R¹¹-(C=O)-)'s, wherein (R¹¹)'s are each independently substituted or unsubstituted C₁-C₂₁ alkyl, substituted or unsubstituted aryl, or substituted or unsubstituted C₁-C₂₁ alkoxy,
      when the R¹¹ is substituted, a substituent is C₁₋₄ alkyl, C₁₋₄ alkoxy, or aryl;
      m and m' are independently an integer of from 2 to 300;
      n is an integer of from 2 to 1,000; and
      20% or more of m or m' (R¹⁰)'s and (R¹⁰')'s are R¹¹-(C=O)-.
(2) The copolymer according to aspect (1), wherein R¹⁰ in formula (I) is R¹¹-(C=O)-, and 100% of m (R¹⁰)'s are (R¹¹-(C=O)-)'s.
(3) The copolymer according to aspect (1), wherein A is defined as (i).
(4) The copolymer according to aspect (1), wherein A is defined as (i), R¹⁰ is R¹¹-(C=O)-, and 100% of m (R¹⁰)'s are (R¹1-(C=O)-)'s.
(5) The copolymer according to aspect (1), wherein A is defined as (ii).
(6) The copolymer according to aspect 1, wherein A is defined as (ii), R¹⁰ and R¹⁰' are (R¹¹-(C=O)-)'s, and 100% of m and m' (R¹⁰)'s and (R¹⁰')'s are (R¹1-(C=O)-)'s.
(7) An ornithine microparticle containing the copolymer described in any one of aspects (1) to (6) and having an average particle diameter of from 1 nanometer to 100 micrometers.
(8) A pharmaceutical composition containing the copolymer described in any one of aspects (1) to (6) as an active ingredient and an additive.
(9) The pharmaceutical composition according to aspect (8), wherein the pharmaceutical composition is an orally administered agent.
(10) A composition for preventing or treating hepatic dysfunction, the composition containing the copolymer described in aspects (1) to (6) or the ornithine microparticle described in aspect (7) as an active ingredient.
(11) A pharmaceutical composition containing the copolymer described in any one of aspects (1) to (6) or the ornithine microparticle described in aspect (7) as an active ingredient and a copolymer represented by formula (IV): 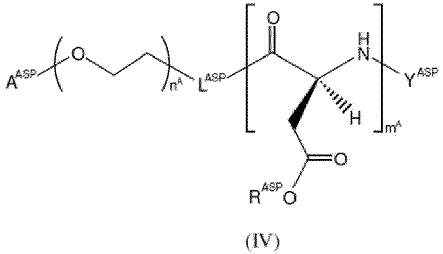 wherein
   A^{ASP} represents a hydrogen atom, an unsubstituted or substituted C₁-C₁₂ alkyl group, an unsubstituted or substituted C₁-C₁₂ alkoxy group, or an unsubstituted or substituted aryl group,
   when the A^{ASP} is substituted, a substituent represents a C₁-C₄ alkyl group, a C₁-C₄ alkoxy group, an aryl group, a formyl group, or a group of formula R₁R²CH- (wherein R¹ and R² independently represent C₁-C₄ alkoxy, or R¹ and R² together represent -OCH₂CH₂O-, - O(CH₂)₃O-, or -O(CH₂)₄O-);
   L^{ASP} represents a linking group;
   R^{ASP} is a hydrogen atom, substituted or unsubstituted C₁-C₂₁ alkyl, or substituted or unsubstituted aryl,
   when the R^{ASP} is substituted, a substituent is C₁₋₄ alkyl, C₁₋₄ alkoxy, or aryl;
   Y^{ASP} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
   m^{A} is an integer of from 2 to 300; and
   n^{A} is an integer of from 2 to 1,000.
(12) A pharmaceutical composition containing the copolymer described in any one of aspects (1) to (6) or the ornithine microparticle described in aspect (7) as an active ingredient and a copolymer represented by formula (V): 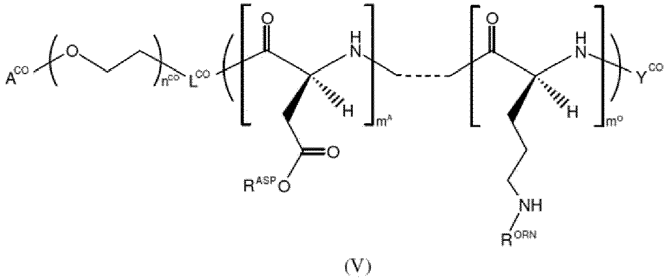 wherein
   A^{CO} represents a hydrogen atom, an unsubstituted or substituted C₁-C₁₂ alkyl group, an unsubstituted or substituted C₁-C₁₂ alkoxy group, or an unsubstituted or substituted aryl group,
   when the A^{CO} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
   n^{CO} is an integer of from 2 to 1,000;
   L^{CO} represents a linking group;
   R^{ASP} is a hydrogen atom, substituted or unsubstituted C₁-C₂₁ alkyl, or substituted or unsubstituted aryl,
   when the R^{ASP} is substituted, a substituent is C₁₋₄ alkyl, C₁₋₄ alkoxy, or aryl;
   m^{A} is an integer of from 2 to 300;
   R^{ORN} is a hydrogen atom or R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
   when the R¹¹ is substituted, a substituent is C₁₋₄ alkyl, C₁₋₄ alkoxy, or aryl;
   m^{O} is an integer of from 2 to 300; and
   Y^{CO} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl;
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino,
   where amino acids in m^{A} repeat units and m^{O} repeat units are each randomly present and form another block.
(13) A pharmaceutical composition containing the copolymer described in any one of aspects (1) to (6) or the ornithine microparticle described in aspect (7) as an active ingredient and a copolymer represented by formula (VI): 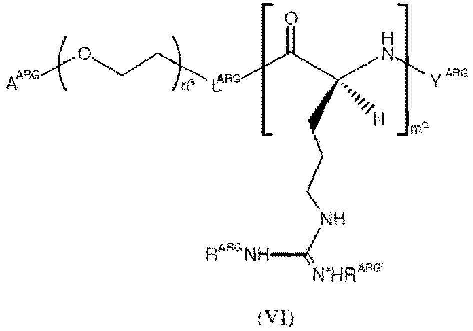 wherein
   A^{ARG} represents a hydrogen atom, a substituted or unsubstituted C₁-C₂₁ alkylcarbonyl group, substituted or unsubstituted arylcarbonyl, or substituted or unsubstituted C₁-C₂₁ alkoxycarbonyl,
   when A^{ARG} is substituted, a substituent is C₁₋₄ alkyl, C₁₋₄ alkoxy, or aryl;
   n^{G} is an integer of from 2 to 1,000;
   L^{ARG} represents a linking group;
   R^{ARG} and R^{ARG'} are independently a hydrogen atom or R¹¹-(C=O)-, wherein R¹¹ are each independently substituted or unsubstituted C₁-C₂₁ alkyl, substituted or unsubstituted aryl, or substituted or unsubstituted C₁-C₂₁ alkoxy,
   when the R¹¹ is substituted, a substituent is C₁₋₄ alkyl, C₁₋₄ alkoxy, or aryl;
   m^{G} is an integer of from 2 to 300; and
   Y^{ARG} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino.
(14) A copolymer represented by Formula (IV-a): 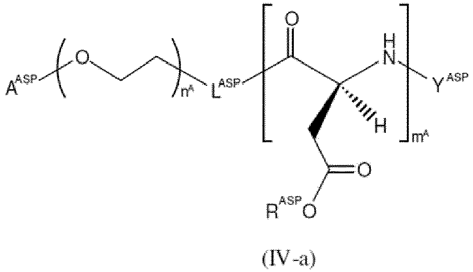 wherein
   A^{ASP} represents a hydrogen atom, an unsubstituted or substituted C₁-C₁₂ alkyl group, an unsubstituted or substituted C₁-C₁₂ alkoxy group, or an unsubstituted or substituted aryl group,
   when the A^{ASP} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
   L^{ASP} represents a linking group;
   Y^{ASP} represents C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residues,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
   R^{ASP} is substituted or unsubstituted C₁-C₂₁ alkyl, or substituted or unsubstituted aryl,
   when the R^{ASP} is substituted, a substituent is C₁₋₄ alkyl, C₁₋₄ alkoxy, or aryl, provided that they are other than benzyl;
   m^{A} is an integer of from 2 to 300; and
   n^{A} is an integer of from 2 to 1,000.
(15) A copolymer represented by Formula (V): 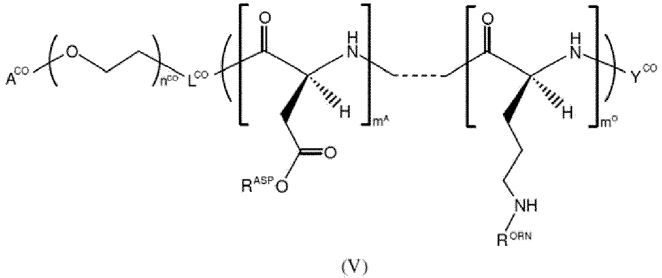 wherein
   A^{CO} represents a hydrogen atom, an unsubstituted or substituted C₁-C₁₂ alkyl group, an unsubstituted or substituted C₁-C₁₂ alkoxy group, or an unsubstituted or substituted aryl group,
   when the A^{CO} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
   n^{CO} is an integer of from 2 to 1,000;
   L^{CO} represents a linking group;
   R^{ASP} is a hydrogen atom, substituted or unsubstituted C₁₋₂₁ alkyl, or substituted or unsubstituted aryl,
   when the R^{ASP} is substituted, a substituent is C₁₋₄ alkyl, C₁₋₄ alkoxy, or aryl;
   m^{A} is an integer of from 2 to 300,
   R^{ORN} is a hydrogen atom or R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
   when the R¹¹ is substituted, a substituent is C₁₋₄ alkyl, C₁₋₄ alkoxy, or aryl;
   m^{O} is an integer of from 2 to 300; and
   Y^{CO} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl;
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino,
   where amino acids in m^{A} repeat units and m^{O} repeat units are each randomly present and form another block.
(16) A copolymer represented by formula (VI-a): 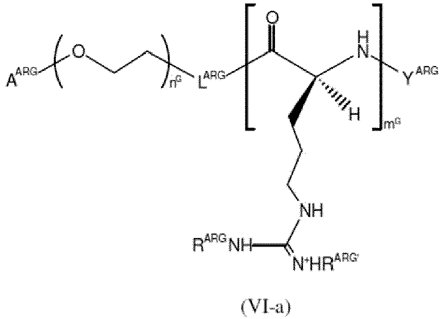 wherein
   A^{ARG} represents a hydrogen atom, a substituted or unsubstituted C₁-C₂₁ alkylcarbonyl group, substituted or unsubstituted arylcarbonyl, or substituted or unsubstituted C₁-C₂₁ alkoxycarbonyl,
   when the A^{ARG} is substituted, a substituent is C₁₋₄ alkyl, C₁₋₄ alkoxy, or aryl;
   n^{G} is an integer of from 2 to 1,000;
   L^{ARG} represents a linking group;
   R^{ARG} and R^{ARG'} are independently R¹¹-(C=O)-, wherein R¹¹ are each independently substituted or unsubstituted C₁-C₂₁ alkyl, substituted or unsubstituted aryl, or substituted or unsubstituted C₁-C₂₁ alkoxy,
   when the R¹¹ is substituted, a substituent is C₁₋₄ alkyl, C₁₋₄ alkoxy, or aryl, provided that they are other than benzyloxycarbonyl and tert-butyloxycarbonyl;
   m^{G} is an integer of from 2 to 300; and
   Y^{ARG} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
   where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
   when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino.

### Advantageous Effects of Invention

The self-assembling drug containing the copolymer of the present invention as an active ingredient is orally administered and used as a novel therapeutic drug for hepatic disorders. In particular, when orally administered, the self-assembling drug accumulates in the intestinal mucosa, is hydrolyzed by a degrading enzyme, and gradually liberates ornithine, resulting in high bioavailability of ornithine in the body and, besides, sustained effect. On the other hand, the toxicity of the self-assembling drug of the present invention is low.

### Brief Description of Drawings

FIG. 1 is a ¹H-NMR chart of a compound synthesized in Production Example 4.
FIG. 2 is a ¹H-NMR chart of a compound synthesized in Production Example 5.
FIG. 3 is a ¹H-NMR chart of a compound synthesized in Production Example 6.
FIG. 4 is a ¹H-NMR chart of a compound synthesized in Production Example 7.
FIG. 5 illustrates results of dynamic light scattering measurement of Nano^{ORN (iBu)}.
FIG. 6 illustrates results of measurements of pH-dependency of particle diameter (left) and scattering intensity (right) in dynamic light scattering measurements of Nano^{ORN (Z)}, Nano^{ORN (Me)}, and Nano^{ORN (iBu)}.
FIG. 7 illustrates evaluation results of blood ammonia concentration (a), ALT (b), and AST of ornithine nanostructures for acetaminophen (APAP) acute hepatic disorder mice.
FIG. 8 includes views of hematoxylin and eosin (H & E) staining of liver tissue after administration of ornithine nano-assembled bodies to APAP acute hepatic disorder mice.
FIG. 9 illustrates changes in body weight after administration of nano-assembled bodies to normal mice.
FIG. 10 illustrates changes in leukocyte count and platelet count after administration of nano-assembled bodies to normal mice.
FIG. 11 is a graph of survival rate after administration of nano-assembled bodies to APAP acute hepatic disorder mice.
FIG. 12 illustrates results of kinetic analysis after oral administration of ¹²⁵I-labeled nano-assembled bodies (Nano^{ORN (iBu)}).
FIG. 13 illustrates results of cytotoxicity evaluation of each polyornithine nano-assembled bodies (Nano^{ORN}).

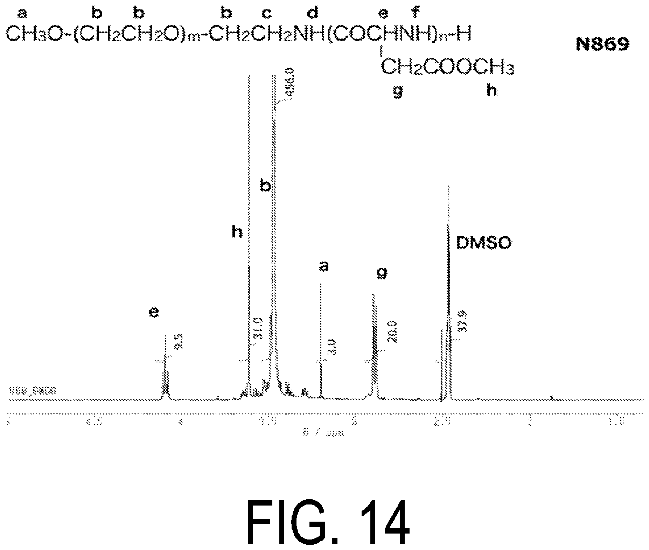

FIG. 14 is a ¹H-NMR chart of a compound synthesized in Production Example 16.

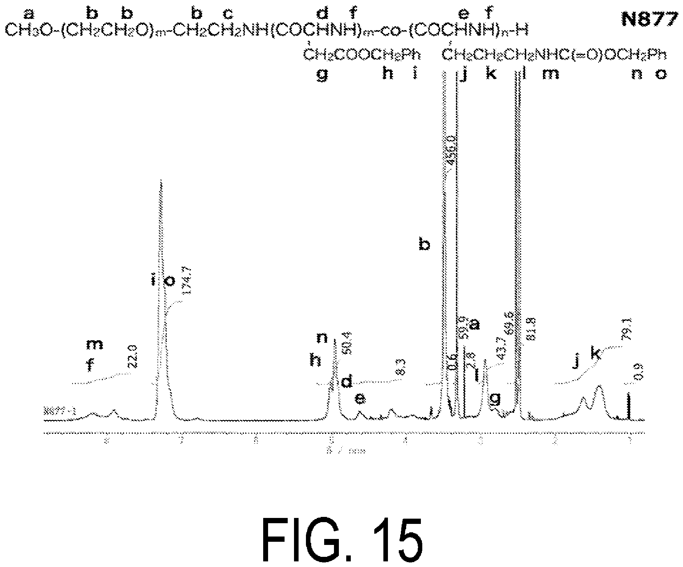

FIG. 15 is a ¹H-NMR chart of a compound synthesized in Production Example 17.

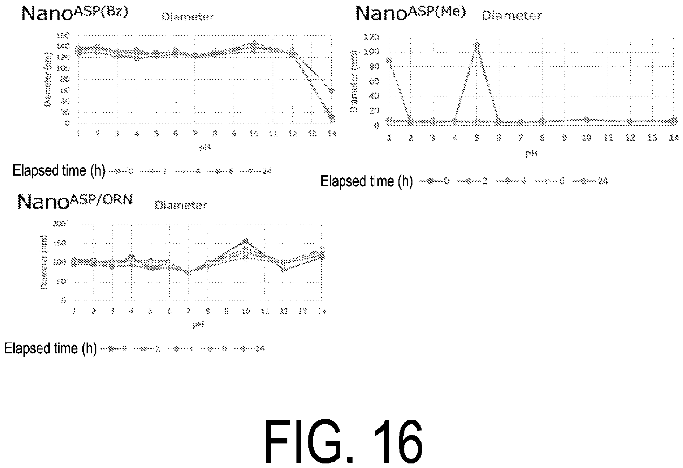

FIG. 16 illustrates results of measurements of pH-dependency of particle diameter in dynamic light scattering measurements of Nano^{ASP (Bz)}, Nano^{ASP (Me)}, and Nano^{ASP/ORN}.
FIG. 17 illustrates evaluation results of blood ammonia concentration (a), ALT (b), and AST of structures such as Nano^{ASP (Bz)}, Nano^{ASP (Me)}, and Nano^{ASP/ORN} for acetaminophen (APAP) acute hepatic disorder mice.
FIG. 18 is a ¹H-NMR chart of a compound synthesized in Production Example 21.
FIG. 19 is a ¹H-NMR chart of a compound synthesized in Production Example 22.
FIG. 20 illustrates results of dynamic light scattering measurements of Nano^{ARG (Ac)} and Nano^{ARG (iBu)}.
FIG. 21 illustrates an effect of acetylated arginine assembled bodies on APAP-induced acute hepatic disorder mice via oral administration.
FIG. 22 illustrates an effect of acetylated arginine assembled bodies on nonalcoholic steatohepatitis (NASH) model mice.

### Description of Embodiments

The technical terms used herein have meanings commonly used in the art, unless otherwise defined.

### <Copolymer>

The copolymer according to an embodiment of the present invention is as represented by formula (I) of aspect (1).

In formula (I), A
(i) represents a hydrogen atom, an unsubstituted or substituted C₁-C₁₂ alkyl group, an unsubstituted or substituted C₁-C₁₂ alkoxy group, or an unsubstituted or substituted aryl group,
   when the A is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-, or
(ii) is a group represented by a formula. The C₁-C₄ alkyl group, C₁-C₄ alkoxy group, and aryl group, which are indicated as substituents may be further substituted with a C₁-C₄ alkyl group, a C₁-C₄ alkoxy group, or an aryl group. The aryl group also includes phenyl, biphenyl and naphthyl.

The linking group(s) L and/or L' in formula (I) can be any organic divalent group(s) as long as it/they does/do not adversely affect the formation of the ornithine microparticles, but generally represent(s) -O-(CH2)ₐ-NH-, -O-(CH2)a-O-, -(CH2)a-NH- or -(CH2)a-O-, preferably -O-(CH₂)ₐ-NH-, -(CH₂)ₐ-NH-, wherein a is an integer of from 1 to 6, preferably from 1 to 3. The bonding directionality of these linking groups is forward directionality of each moiety in the structural formula of each formula with respect to L. For example, taking -O-(CH₂)ₐ-NH- as an example, the linker on the -O-(CH₂) moiety side is covalently bonded to the methylene of formula (I), and the NH- moiety is covalently bonded to the carbonyl group. On the other hand, the directionality of L' is opposite to that of L.

In formula (I), Y and Y' each independently represent a hydrogen atom, unsubstituted C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl.

The alkyl as each group or a part of each group above can be straight or branched chain, and can be, but is not limited to, applicable one among methyl, ethyl, propyl, iso-propyl, butyl, sec-butyl, tert-butyl, pentyl, hexyl, heptyl, nonyl, undecyl, tridecyl, heptadecyl, nonadecyl and the like. Preferred aryl is selected from C₁₋₆ alkyl. The C₃₋₇ cycloalkyl can be cyclopropyl, cyclobutyl, cyclopentyl, cyclohexyl, or cycloheptyl. The aryl can be phenyl, biphenyl, or naphthyl. The 5- or 6-membered heteroaryl is an unsaturated heterocyclic radical containing one or two identical or different heteroatoms selected from oxygen, nitrogen and sulfur atoms, and may be thienyl, furyl, pyranyl, pyrrolyl, isoxazole, pyrazolyl, imidazolyl, pyridyl, pyrazinyl, or pyrimidinyl, and these heterocyclic rings may also be benzo-fused. Such fused rings include, for example, isoindolyl, indolyl, quinolyl, isoquinolyl, quinoxalinyl, quinazolinyl, and phenanthridinyl.

The substituent of the substituted C₁₋₄ alkylcarbonyl is selected from the group consisting of halogen atoms (Cl, F, Br, and I), hydroxyl, carboxyl, unsubstituted or substituted C₃₋₇ cycloalkyl, unsubstituted or substituted aryl and unsubstituted or substituted 5- or 6-membered heteroaryl, unsubstituted or substituted adamantyl, and unsubstituted or substituted cholesterol residues. When the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino. The last substituent is also applied to substituted C₃₋₇ cycloalkylcarbonyl, substituted arylcarbonyl, or substituted 5- or 6-membered heteroarylcarbonyl.

In the above definitions, the cholesterol residue can be a residue in which any H on the 22nd to 27th carbons in the cholesterol molecule is eliminated or a hydrocarbon chain containing any of the 22nd to 27th carbons is eliminated. Examples of the substituted alkylcarbonyl of such a residue include cholic acid and chenodeoxycholic acid. Preferred as Y is C₁₋₆ alkylcarbonyl.

R¹⁰ and R¹⁰' in formulae (I) and (ii) are independently a hydrogen atom or R¹¹-(C=O). Without being bound by theory, when R¹⁰ and R¹⁰' include R¹¹-(C=O), it is assumed that the amide bond between this and the δ-amino group of the polyornithine segment and the amide (or peptide) bond of the polyornithine main chain are each subjected to enzymatic degradation, resulting in release of free ornithine. Also, it is assumed that they are degraded, for example, by digestive enzymes in the digestive tract, resulting in gradual release of ornithine.

R¹¹ is substituted or unsubstituted C₁-C₂₁ alkyl, substituted or unsubstituted aryl, or substituted or unsubstituted C₁-C₂₁ alkoxy. When the R¹¹ is substituted, a substituent is C₁₋₄ alkyl, C₁₋₄ alkoxy, or aryl, and, more preferably, R¹¹ can be a methyl, ethyl, propyl, iso-propyl, butyl, iso-butyl, sec-butyl, tert-butyl, (optionally branched) pentyl, (optionally branched) hexyl, (optionally branched) heptyl, (optionally branched) octyl, cyclopropyl, cyclobutyl, cyclopentyl, cyclohexyl, cycloheptyl, phenyl, naphthyl, benzyloxy, or tert-butyloxy group. These groups are preferable from the perspective of efficiency in assembling the copolymer and forming the ornithine microparticles. On the other hand, from the perspective of ease of synthesis, R¹¹ is preferably a methyl, propyl, isopropyl, butyl, isobutyl, phenyl (which may have a substituent), or benzyl (which may have a substituent) group.

For example, when R¹¹ is methyl, it can be obtained by using, for example, acetic anhydride as a carboxylic acid ester in a condensation reaction of formula (II) which will be described later in the "production of the copolymer" section. Similarly, the desired R¹¹ can be obtained by changing the kind of carboxylic acid, for example, using propionic anhydride when R¹¹ is ethyl, using butyric anhydride when R¹¹ is propyl, using isobutyric anhydride when R¹¹ is isopropyl, using pentanoic anhydride when R¹¹ is butyl, using isopentanoic anhydride when R¹¹ is isobutyl, using benzoic anhydride when R¹¹ is phenyl, and using bisphenylacetic anhydride when R¹¹ is benzyl. By using a carboxylic acid ester having a substituent or a derivative thereof, R¹¹ can also have a substituent.

m and m' may independently be an integer of preferably from 2 to 300, more preferably from 15 to 150, and most preferably from 15 to 100, from the perspective of stability of particles formed by the copolymer.

Similarly, n can be an integer of preferably from 2 to 1000, more preferably from 10 to 500, and most preferably from 30 to 400.

The m and m' (R¹⁰)'s and (R¹⁰')'s in formula (I) can independently be hydrogen atoms in a proportion of generally up to 80%, preferably up to 60%, more preferably up to 30%, most preferably up to 10%, and, particularly most preferably, all (100%) of the m and m' (R¹⁰)'s and (R¹⁰')'s are (R¹¹-(C=O))'s.

### <Production of copolymer>

An example of a scheme for synthesizing the copolymer will be shown below. However, the following is merely an example, and the present invention is not limited by the example.

The copolymer may contain the corresponding PEG segment and polyornithine segment produced by any method as long as it is consistent with the objectives of the present invention. However, the copolymer is preferably a copolymer having a narrow molecular weight distribution and capable of providing ornithine microparticles formed by self-assembly in an aqueous medium with an appropriate average diameter.

The copolymer can be provided by preparing a copolymer represented by the following formula (II) in advance, and then adding a compound such as a carboxylic acid ester for the purpose of introducing an acyl group into the δ-amino group in the segment derived from L-ornithine in the formula. 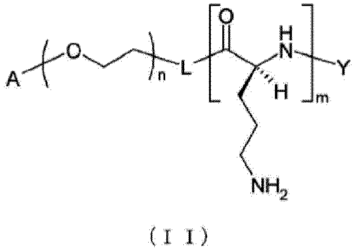 In the formula,
A represents:
(i)' a hydrogen atom, an unsubstituted or substituted C₁-C₁₂ alkyl group, an unsubstituted or substituted C₁-C₁₂ alkoxy group, or an unsubstituted or substituted aryl group,
when the A is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-), or
(ii)' formula: 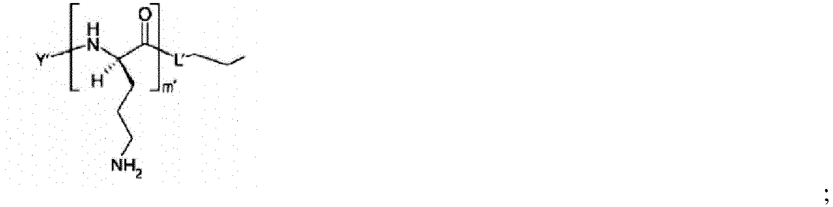
L and L' independently represent a linking group;
Y and Y' independently represent a hydrogen atom, C₁-C₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
m and m' are independently an integer of from 2 to 300; and
n is an integer of from 2 to 1,000.

A typical example of such production is a condensation reaction between the copolymer represented by formula (II) and a carboxylic acid ester as shown in the following scheme.

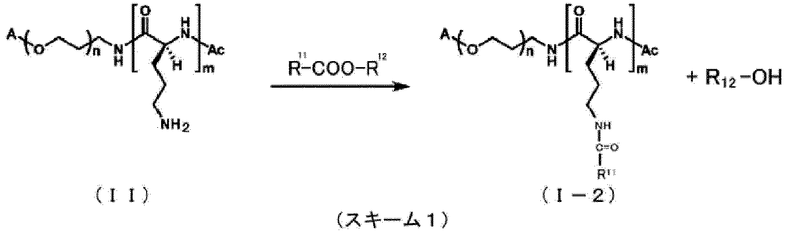

Examples of the carboxylic ester include carboxylic halides, carboxylic anhydrides, carboxylic azides and active esters, and specific examples thereof include acetic, propionic, isobutyric, butyric, oxalic, succinic, glutaric, adipic, maleic, phthalic, benzoic, and bisphenylacetic anhydrides.

When A in formula (II) in the above reaction scheme corresponds to that defined in (ii)' above, the δ-amino group in the segments derived from ornithine corresponding to the m' repeat units in the formula described therein undergoes a condensation reaction in the same manner as that of the m repeat unit.

Further, for the purpose of providing the copolymer according to an embodiment of the present invention having a narrow molecular weight distribution, the precursor copolymer represented by formula (II) (taking the case where A in formula (II) is represented by (I)' as an example) is preferably produced according to the following synthesis scheme.

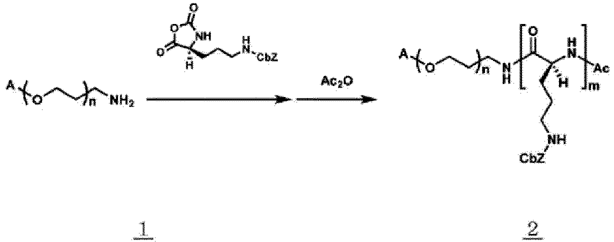

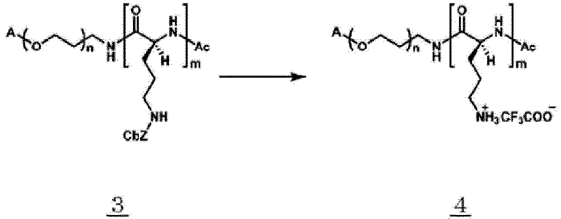

Here, compound 1 is commercially available or prepared by a method according to its provision method and has a molecular weight distribution as narrow as possible; compound 2 is produced by subjecting the compound to living ring-opening polymerization with N-carboxylic anhydride of ornithine in which the amino group at the δ-position is protected, and then terminating the reaction using a living terminal modifier such as acetic anhydride by a method known per se; and further an amino protecting group in the poly(L-ornithine) segment is protected.

According to such a reaction treatment using the compound 1 as a raw material, the poly(L-ornithine) segment can also be provided as a precursor copolymer having an extremely narrow molecular weight distribution.

Therefore, the finally obtained block copolymer represented by formula (I) according to an embodiment of the present invention has a molecular weight distribution of from 1.01 to 1.20, preferably from 1.01 to 1.06.

On the other hand, when A in formula (II) is defined as (ii)', the compound 1 as the starting material in the above reaction scheme may be NH₂CH₂CH₂-(OCH₂CH₂)ₙ-NH₂, hereinafter, the A moiety of each of compounds 2 to 4 may be repeat units corresponding to the m repeat units. The triblock copolymer thus obtained, in which A in formula (II) is defined as (ii)', is represented by the following formula (III): 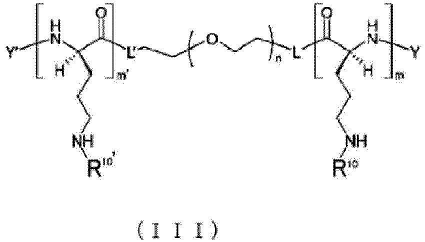 wherein L, L', Y, Y' and m, m', and n are as defined for formula (II) above.

As another example, the copolymer can also be synthesized by a synthesis method as represented by the following scheme, starting from the compound 1 described above.

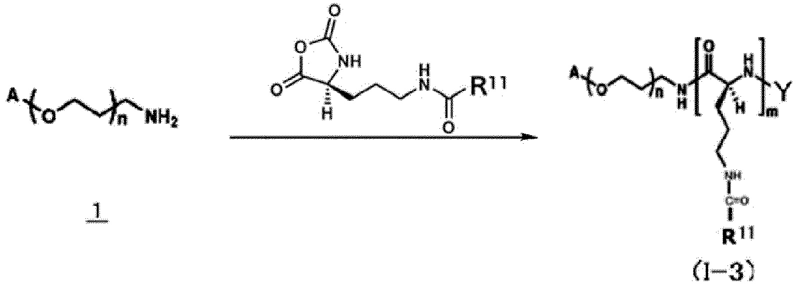

### <Ornithine microparticle>

The ornithine microparticles can be produced by assembling the copolymer represented by formula (I) in water or an aqueous medium (which may contain a water-soluble organic solvent or a buffer). It is considered that, since the polyornithine segment containing R¹⁰ and R¹⁰' exhibits hydrophobicity, the copolymer aggregates and self-assembles, and forms ornithine microparticles having the polyornithine segment as a core and the PEG segment as a shell. For example, ornithine microparticles can be obtained by dissolving the copolymer represented by formula (I) in a polar organic solvent such as dimethylformamide (DMF) or dimethyl sulfoxide (DMSO) and dialyzing the solution in water, an aqueous solution or a buffer solution.

The ornithine microparticles (or assembled bodies) are not particularly limited, but from the perspective of ease of administration or production, the average particle diameter is preferably 1 millimeter or less, more preferably from 1 nanometer to 1 millimeter, and still more preferably from 1 nanometer to 100 micrometers. More preferably, it is from 10 to 200 nanometers, and most preferably from 20 to 70 nanometers. The particle diameter can be measured by dynamic light scattering (DLS) or the like in water or an aqueous medium.

For example, polyion complex particles (PIC) can be obtained by adding a solution of a polyanionic compound polymer such as chondroitin to compound 3 or formula (II) and thus forming a complex thereof. On the other hand, the ornithine microparticles of the present invention are characterized in that they are obtained by self-assembly of a poly(ethylene glycol)-b-poly(ornithine) copolymer or a derivative thereof alone in an aqueous medium and are not formed into a complex with another polyanionic compound polymer. Therefore, the ornithine microparticles of the present invention are distinguished from PIC.

Thus, preferably, the ornithine particles of the present invention is characterized in that they contain a poly(ethylene glycol)-b-poly(omithine) polymer or a derivative thereof as a main component, and do not contain a polyanionic polymer or the like. Examples of the polyanionic polymer can include polyacrylic acid, polymethacrylic acid, polysulfonic acid, polyanionic polysaccharide, and anionic protein, and, more specifically, can include chondroitin sulfate, carrageenan, heparin, carboxymethyl dextran, xanthan gum, hyaluronic acid, polyaspartic acid, polyglutamic acid, polyacrylic acid, polymethacrylic acid, polyvinyl sulfate, and polystyrene sulfonic acid. However, the ornithine microparticles of the present invention may contain an aqueous medium, a solvent or the like as long as the stability of the particles is not adversely affected.

The obtained ornithine microparticles can be separated by a separation means such as centrifugation, or can be stored as a dry composition by freeze-drying, and can be reconstituted in an aqueous medium as needed. Such a dry composition can be provided as an aqueous solution of ornithine microparticles optionally containing a physiologically acceptable diluent or excipient. Such a diluent may be sterile water, physiological saline, a solution containing a physiologically acceptable buffer, or the like, and may further contain, as an additive, for example, sorbitol, dextrin, glucose, mannitol, an amino acid (for example, glycine, isoleucine, valine, methionine, glutamic acid, or the like), or the like.

In other words, as described above, one embodiment of the present invention provides the following method for producing ornithine microparticles. That is, the present invention provides a method for producing ornithine microparticles, including reacting a copolymer represented by the above formula (II) with a carboxylic acid ester compound; adding a substituent composed of R¹⁰ to a δ-amino group in a segment derived from ornithine, wherein R¹⁰ is R¹¹-(C=O)-, R¹¹ is substituted or unsubstituted C₁-C₂₁ alkyl, substituted or unsubstituted aryl, or substituted or unsubstituted C₁-C₂₁ alkoxy, and when these substituents are substituted, substituents are C₁₋₄ alkyl, C₁₋₄ alkoxy, or aryl; and assembling the resulting copolymer in water.

### <Further copolymers which can also be used as other additives together with or independently of additives described above or below (which may include novel compound)>

The copolymer represented by formula (I) or the ornithine microparticles from the copolymer may be provided as pharmaceutical compositions containing the following copolymer together with or independently of those described above as additives, for example, pharmaceutical compositions for preventing or treating hepatic dysfunction.
- Copolymer represented by formula (IV):
   In the formula, A^{ASP} and L^{ASP} have the same definitions as A (i) and L in formula (I), and specifically, the description for L in formula (I) can be applied. Y^{ASP} has the same definition as Y in formula (I). Further, R^{ASP} is a hydrogen atom, substituted or unsubstituted C₁-C₂₁ alkyl, or substituted or unsubstituted aryl, and when these substituents are substituted, substituents are C₁₋₄ alkyl, C₁₋₄ alkoxy, or aryl. With respect to the C₁-C₂₁ alkyl and aryl, the description for R¹¹ in formula (I) can be applied. Specific examples of the linking group as L^{ASP} are the same as those specifically described for L.
   m^{A} is an integer of from 2 to 300, preferably from 15 to 150, more preferably from 15 to 100, and
   n^{A} is an integer of from 2 to 1,000, preferably from 10 to 500, and more preferably from 30 to 400.
- As the copolymer represented by formula (IV), a compound in which m protected ornithine units in the compound 2 are replaced with protected aspartic acid units can be provided by living ring-opening polymerization of the omega (ω) terminal amino group of compound 1 using, for example, N-carboxylic anhydride of benzylated or alkylated β-carboxyl of aspartic acid instead of N-carboxylic anhydride of ornithine having a protected δ-amino group in a reaction scheme from the compound 1 to compound 4. The compound thus provided can be obtained, if necessary, through protective group elimination, esterification, alkyl group exchange reaction, and the like which are known per se. When R^{ASP} is a hydrogen atom or benzyl, compounds known per se may be included, but other compounds represented by formula (IV-a) are considered to be copolymers which are not described in prior art documents as far as the present inventors know. Such copolymers can also be automatically assembled in an aqueous medium and form microparticles having a size similar to that of the copolymer represented by formula (I).

Since low-molecular-weight L-aspartic acid also has an action of discharging harmful ammonia similar to low-molecular-weight L-ornithine to the outside of the body, the copolymer represented by formula (IV) can also enhance the action or effect of the copolymer of formula (I), or can enhance the stability or the like of a pharmaceutical composition containing the copolymer of formula (I) or microparticles (assembled bodies) thereof.
- Copolymer represented by formula (V):
   In the formula, A^{CO}, L^{CO}, n^{CO}, R^{ASP}, and m^{A} have the same meanings as A^{ASP}, L^{ASP}, n^{A}, R^{ASP}, and m^{A}, respectively, defined for formula (IV) above. A^{ORN}, R^{ORN}, m^{O} and Y^{CO} have the same definitions as A (i), R, m, n and Y, respectively, defined for formula (I). In this copolymer, the respective m^{ASP} units and the respective m^{ORN} units can be each present at random.
   The copolymer represented by formula (V) can be provided as a polymer in which m protected ornithine units in the compound 2 are randomly replaced with protected aspartic acid units and protected ornithine units, by living ring-opening polymerization of N-carboxylic anhydrides to the omega (ω) terminal amino group of the compound 1 and the newly generated amino group in the reaction scheme from the compound 1 to the compound 4 using, for example, a mixture of N-carboxylic anhydride of benzylated β-carboxyl of aspartic acid and N-carboxylic anhydride of protected ornithine instead of N-carboxylic anhydride of δ-amino group-protected ornithine, and can be obtained through protective group elimination, esterification and/or acylation known per se. The copolymer thus provided can also be assembled similarly to the copolymer of formula (I).
   The copolymer represented by formula (V) can also have the same action and effect as those of the copolymer represented by formula (I) or (IV) and the assembled product thereof.
- Copolymer represented by formula (VI):
   Wherein A^{ARG}, L^{ARG}, n^{G}, m^{G}, and Y^{ARG} have the same meanings as A^{ASP}, L^{ASP}, n^{A}, m^{A}, and Y^{ARG}, respectively, defined for formula (IV) above, and R^{ARG} and R^{ARG'} independently have the same meanings as R^{ASP defined for formula (IV) above.} The copolymer of formula (IV) can conveniently be produced by a reaction of the copolymer described in WO2016/167333 A1 wherein R^{ARG} and R^{ARG'} are each H and Y^{ARG} may have the same definition as Y^{ASP} in formula (IV) with anhydrides or carboxylic acids capable of forming R^{ARG} and R^{ARG'}. The copolymers thus prepared can be assembled without the use of polyanionic polymers. These copolymers or assembled products thereof can have the same actions and effects as the copolymers of formula (I) and assembled products thereof, except that they have been confirmed to exhibit anti-tumor effects on lung cancer-metastasis model mice and subcutaneously transplanted cancer-model mice upon oral administration and to greatly reduce the adhesion of melanoma cells to the lungs (data unpublished), in addition to the actions and effects (anti-tumor effects) described in WO2016/167333 A1.

### <Composition for preventing or treating hepatic dysfunction>

The ornithine microparticles (or assembled products) formed from the copolymer of formula (I) according to an embodiment of the present invention are used as a composition for the prevention and treatment of hepatic disorders. That is, as one embodiment, the present invention provides a pharmaceutical composition for use in preventing or treating hepatic dysfunction, which contains the ornithine microparticles according to an embodiment of the present invention as an active ingredient.

Ornithine functions as a substrate for ureagenesis or activates urea cycle enzymes including carbamylphosphate synthase (CPS) and ornithine transcarbamylase (OTC). Through these actions, effects of prevention and treatment of hepatic disorders are expected.

When orally administered, the ornithine microparticles according to an embodiment of the present invention accumulate in the intestinal mucosa, gradually undergo enzymatic degradation, and cause hydrolysis of the amide bond in the polymer, resulting in release of ornithine. Therefore, the ornithine microparticles effectively act in the living body, have high bioavailability, and are very effectively used in the prevention or treatment of hepatic dysfunction as shown in model animal experiments in the Examples.

The hepatic dysfunction includes viral liver disease, drug- and alcohol-related liver disease, immune-mediated liver disease, metabolic liver disease, nonalcoholic fatty liver disease (NAFLD), nonalcoholic steatohepatitis (NASH), hepatic failure, fulminant hepatic failure, hepatocellular carcinoma, and liver transplantation complications.

The pharmaceutical composition according to an embodiment of the present invention, ornithine microparticles or an aqueous solution thereof, can be orally administered to a mammal, particularly a human, in need thereof. Since the pharmaceutical composition according to an embodiment of the present invention has low toxicity, is easily accumulated in the liver by injection or the like, and is hydrolyzed there, resulting in release of ornithine, it can be used by direct administration to a vein, an artery, a subcutaneous site, an abdominal cavity or the like, but is more advantageously used as an orally administered agent because it more efficiently exerts its effect in the intestine.

Examples of the dosage form of the pharmaceutical composition include liquid preparations (including solutions for internal use, suspensions, emulsions, and syrups), solid preparations (including tablets, pills, sublingual tablets, capsules, drops, and troches), granules, powders, and powdered drugs. If necessary, the solid preparation can be further formulated into a dosage form coated with a coating known in the art, for example, a sugar-coated tablet, a gelatin-coated tablet, an enteric coated tablet, a film-coated tablet, a double tablet or a multilayer tablet. The shape and size of each dosage form may be within the range of dosage forms known in the art, and additives such as solubilizers, pH adjusters, carriers, excipients, diluents, binders, disintegrators, lubricants, emulsifiers, extenders, colorants, flavoring agents, sweeteners, stabilizers, and preservatives can be contained.

The dose and administration frequency are applied in an optimal concentration range depending on the age, sex, condition, degree of disease, and the like of the patient, but an amount of ornithine to be administered per day is preferably from about 10 µmg to about 1 g, more preferably from 0.1 mg to 100 mg per Kg of body weight, and this amount may be divided into about 1 to 3 times. However, since the pharmaceutical composition agent according to an embodiment of the present invention has high availability, the effect can be expected even if it is administered once a day.

With respect to the dose, administration method, and administration frequency, those skilled in the art can determine an optimum method through animal experiments or experimental administration to humans with reference to various literatures and the like.

### Examples

Hereinafter, the present invention will be described in more detail with reference to specific examples, but is not limited to these examples.

### Production Example 1: Synthesis of CH₃O-(CH₂CH₂O)n-CH₂CH₂OSO₂CH₃ (N813)

To commercial CH₃O-(CH₂CH₂O)ₙ-H (MW = 5,000, 60 g, 12 mmol), THF (200 mL) and 14.4 mL of butyllithium (23 mmol, 1.6 M-hexanes) were added. Then, methanesulfonyl chloride (CH₃SO₂Cl) (1.5 mL, 2.3 g, 20 mmol) was added, and they were reacted at 40°C for 1 day. After precipitation in 2-propanol (IPA), the precipitate is dissolved in methanol for precipitation. This operation was repeated twice, and the precipitate was dried under reduced pressure to give a target product (yield: 60 g).

### Production Example 2: Synthesis of CH₃O-(CH₂CH₂O)n-CH₂CH₂NH₂ (N819)

The CH₃O-(CH₂CH₂O)ₙ-CH₂CH₂OSO₂CH₃ (60 g) obtained in Production Example 1 was added with 600 mL of 28% aqueous ammonia, and they were reacted at 50°C for 1 day. Then, the reaction product was extracted with 50 mL of chloroform, and the chloroform phase was dehydrated with NaHSO₄ and filtered, followed by precipitation in 2-propanol (IPA), and the obtained precipitate was dissolved in methanol for precipitation. This operation was repeated twice, and the precipitate was dried under reduced pressure to give a target product (yield: 54 g).

### Production Example 3: Synthesis of L-ornithine (Z)-N-carboxylic anhydride (L-Orn (Z)-NCA) (N831)

Boc-omithine (Z)-OH (Boc-Orn (Z)-OH, 15 g) was dissolved in 100 mL of THF, and ice-cooled to 0°C, and added with a solution of thionyl chloride (SOCl₂, 10 mL, 16 g) in THF (50 mL), and they were reacted at 0°C for 1 hour and at room temperature for 3 hours. The reaction solution was poured into 1 L of hexane, and the precipitate was filtered, dissolved in 100 mL of ethyl acetate, precipitated in 1 L of hexane, and filtered. This operation was repeated twice, and the resulting precipitate was dried under reduced pressure to give a target product (yield: 10 g).

### Production Example 4: Synthesis of CH₃O-(CH₂CH₂O)n-CH₂CH₂NH-(COCH(CH₂CH₂CH₂NH(Z))NH)m-H (N857)

N819 (5 g) obtained in Production Example 2 was dissolved in 30 mL of DMF, and a solution of N831 (5 g) obtained in Production Example 3 in DMF (20 mL) was added thereto, followed by stirring at room temperature for 2 days. The solution was added to hexane:2-propanol (8:2, 500 mL), and centrifuged to give a polymer, which was dried under reduced pressure to give a target product (6.3 g).

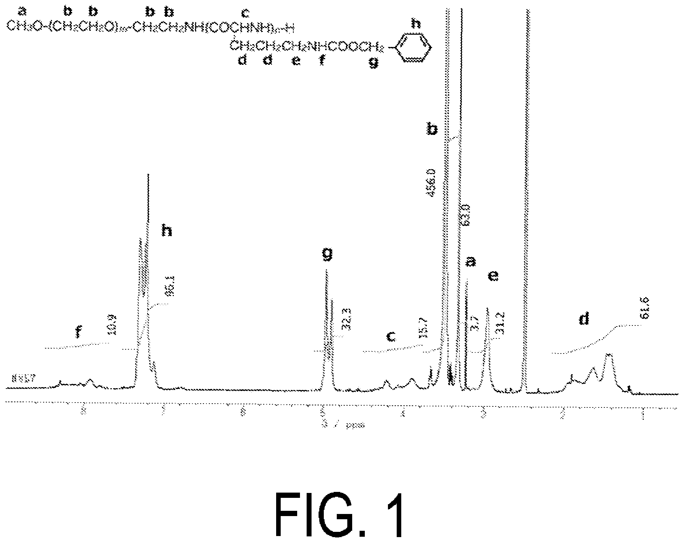

NMR measurement results of the obtained N857 are illustrated in FIG. 1.

### Production Example 5: Synthesis of CH₃O-(CH₂CH₂O)n-CH₂CH₂NH-(COCH(CH₂CH₂CH₂NH₂)NH)m-H (PEG-b-POrn); N967)

N833 (4.3 g) obtained in Production Example 4 was dissolved in 45 mL of trifluoroacetate, ice-cooled to 0°C, and added with 3% HBr (acetic acid solution, 15 mL), and they were reacted for 4 hours. This was added to cold 2-propanol of 800 mL to give a precipitate, which was dried under vacuum to give a target product (yield: 3.3 g).

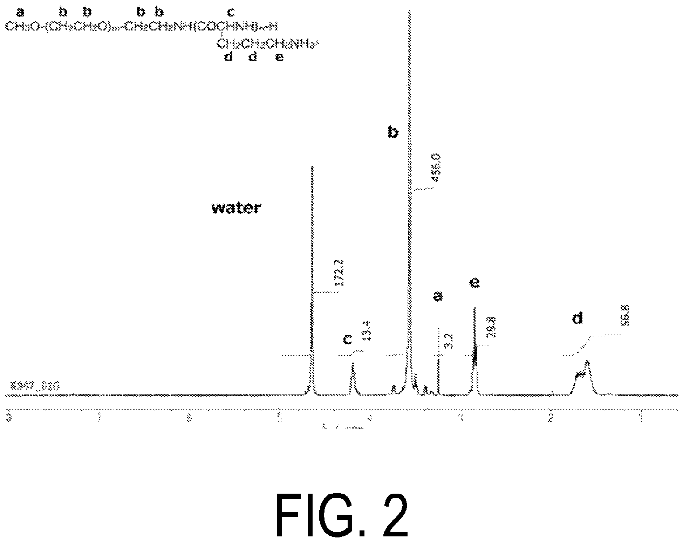

NMR measurement results of the obtained N967 are illustrated in FIG. 2.

### Production Example 6: Synthesis of CH₃O-(CH₂CH₂O)n-CH₂CH₂NH-(COCH(CH₂CH₂CH₂NHC(=O)CH₃)NH)m-C(=O)CH₃ (PEG-b-POrn(COCH₃); N977)

N967 (1 g) obtained in Production Example 5 was dissolved in 1 mL of DMF, and added with 5 mL of triethyl amine and 10 mL of acetic anhydride, and they were reacted at room temperature for 1 hour. This was added to hexane:2-propanol (8:2, 500 mL), and centrifuged to give a polymer, which was dried under reduced pressure to give a target product (1.0 g).

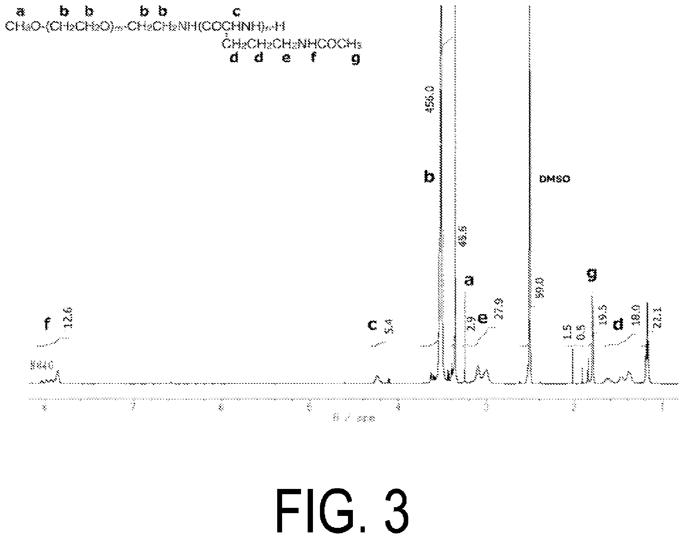

NMR measurement results of the obtained N977 are illustrated in FIG. 3.

### Production Example 7: CH₃O-(CH₂CH₂O)n-CH₂CH₂NH-(COCH(CH₂CH₂CH₂NHC(=O)CH(CH₃)₂)NH)m-C(=O)CH(CH₃)₂ (PEG-b-POrn(COCH(CH₃)₂); N841)

A target polymer (1.4 g) was obtained in exactly the same manner as in Production Example 6, except that isobutyric anhydride was used instead of acetic anhydride.

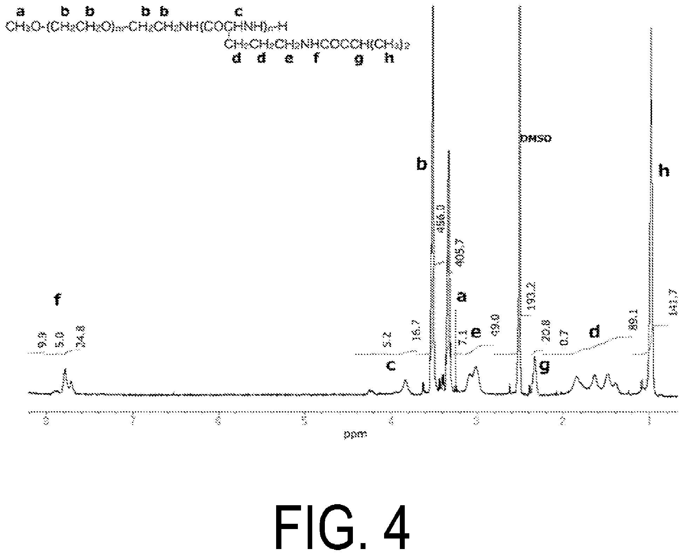

NMR measurement results of the obtained N841 are illustrated in FIG. 4.

### Production Example 8: Preparation of polyion complex particle (Nano^{ORN}) of CH₃O-(CH₂CH₂O)n-CH₂H₂NH-(COCH(CH₂CH₂CH₂NH₂)NH)m-H and chondroitin sulfate

PEG-b-POrn (N836, 1 g) prepared in the same manner as in Production Example 5 was dissolved in 20 mL of DMF, and added with 20 mL of water, the solution was placed in a dialysis membrane (molecular weight cut off (MWCO) = 3.5 KDa), and dialyzed against 0.01 M HCl (2 L) for 24 hours. Thereafter, dialysis was performed for 48 hours against 2 L of distilled and ion-exchanged (DI-water) (dialysis water was exchanged every 12 hours) to give an aqueous solution (85 mL). The total amount of cationic charges in the aqueous solutions was calculated, and a chondroitin sulfate solution (2.7 mg/mL, 115 mL) was added for attaining a ratio of the total amount of cationic charges to the total amount of anionic charges of 1:1. Then, the mixed solution was stirred for 30 minutes to give polyion complex (PIC) particles (Nano^{ORN}).

### Production Example 9: Preparation of PEG-b-POrn (Z) assembled body (Nano^{ORN (Z)})

N833 (1 g) synthesized in Production Example 4 was dissolved in 20 mL of DMF, 20 mL of DI-water was added, the solution was placed in a dialysis membrane (molecular weight cut off (MWCO) = 12 KDa - 14 KDa), and dialyzed against 2 L of DI-water 2L for 72 hours (dialysis water was exchanged every 12 hours) to give an aqueous solution.

### Production Example 10: Preparation of PEG-b-POrn(COCH₃) assembled body (Nano^{ORN (Me)})

This assembled body was made in exactly the same manner as in Production Example 9 except that the N977 (1 g) synthesized in Production Example 6 was used.

### Production Example 11: Preparation of PEG-b-POrn(COCH(CH₃)₂) assembled body (Nano^{ORN (iBu)}) (N978)

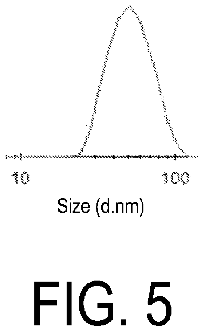

This assembled body was made in exactly the same manner as in Production Example 9 except that the N841 (1 g) synthesized in Production Example 7 was used. Results of dynamic light scattering measurement of the assembled body (Nano^{ORN (iBu)}) are illustrated in FIG. 5.

### Test Example 1: Stability of nano-assembled body

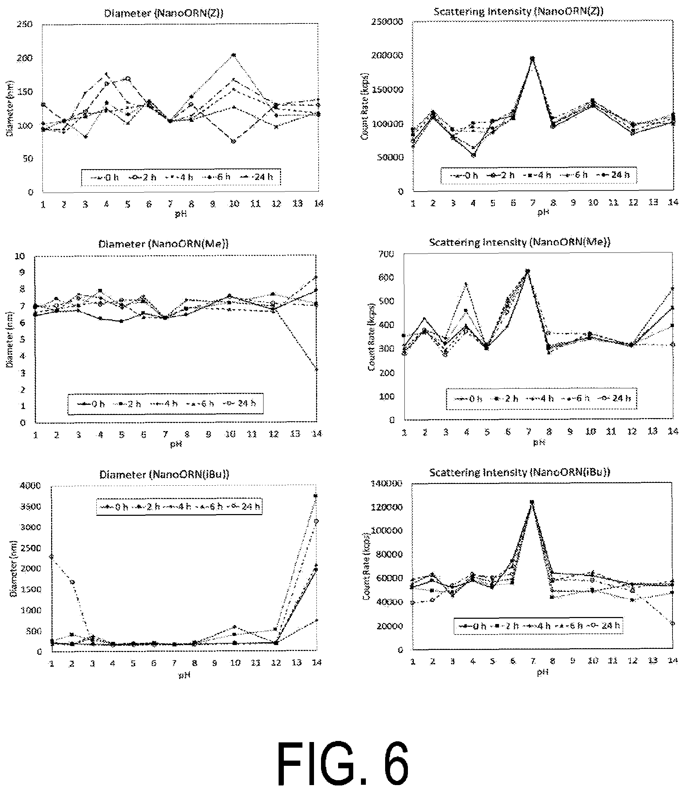

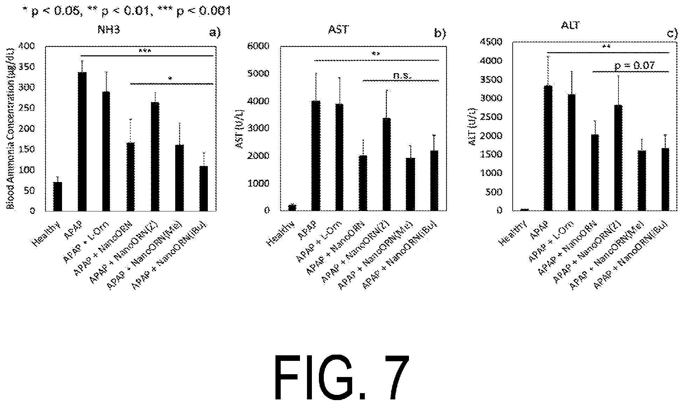

Changes in size of the nano-assembled bodies prepared in Production Examples 9 to 11 depending on pH are illustrated in FIG. 6. All the particles are stable with no change in size or scattering intensity, up to about the pH12. In FIGS. 6 and 7 and other descriptions, the XX portion of Nano^{XX} may be described as NanoXX instead of the superscript expression, but it has the same meaning as the superscript expression.

### Test Example 2: Effect of oral administration of ornithine assembled body to acetaminophen (APAP)-induced acute hepatic disorder mice

Six C57BL/6N mice (male) per group were allowed to freely drink the following samples (for groups 3 to 7, the ornithine content was adjusted to about 200 mg/kg). Three days after the start of administration, an aqueous APAP solution (15 mg/mL, 0.5 mL, 300 mg/Kg-BW) was intraperitoneally administered. After 24 hours, blood and livers were collected, whole blood were used for measurement of a blood ammonia concentration, plasmas were used for measurement of hepatic disorder markers AST and ALT, and livers were subjected to hematoxylin-eosin staining for histological diagnosis.
Group 1: healthy group
Group 2: APAP-administered group
Group 3: APAP-administered group/allowed to freely orally take 2 mg/mL L-ornithine
Group 4: APAP-administered group/allowed to freely orally take 5 mg/mL Nano^{ORN}
Group 5: APAP-administered group/allowed to freely orally take 5 mg/mL PEG-b-POrn (Z) assembled body (Nano^{ORN (Z)})
Group 6: APAP-administered group/freely orally drinking 5 mg/mL PEG-b-POrn(COCH₃) assembled body (Nano^{ORN (Me)})

### Water free intake

Group 7: APAP-administered group/freely orally drinking 5 mg/mL PEG-b-POrn(COCH(CH₃₎₂) assembled body (Nano^{ORN (iBU)}

As illustrated in FIG. 7a, the blood ammonia concentration was greatly increased in the APAP-administered group as compared with the healthy group, and the low-molecular-weight ornithine could not reduce it. It was confirmed that Nano^{ORN} was somewhat effective, but that the PEG-b-POm(COCH(CH₃)₂ assembled body (Nano^{ORN (iBu)}) significantly reduced it as compared with Nano^{ORN}.

FIGS. 7b and 7c illustrate blood aspartate aminotransferase (AST) and alanine aminotransferase (ALT) levels. Both values were significantly increased by APAP administration. This indicates the development of a hepatic disorder. Both of the AST and ALT levels were decreased upon APAP administration in Nano^{ORN} (PIC: ion complex), PEG-b-POrn(COCH₃) assembled body (Nano^{ORN(ME)}) and PEG-b-POrn(COCH(CH₃)₂) assembled body (Nano^{ORN(iBu)}).

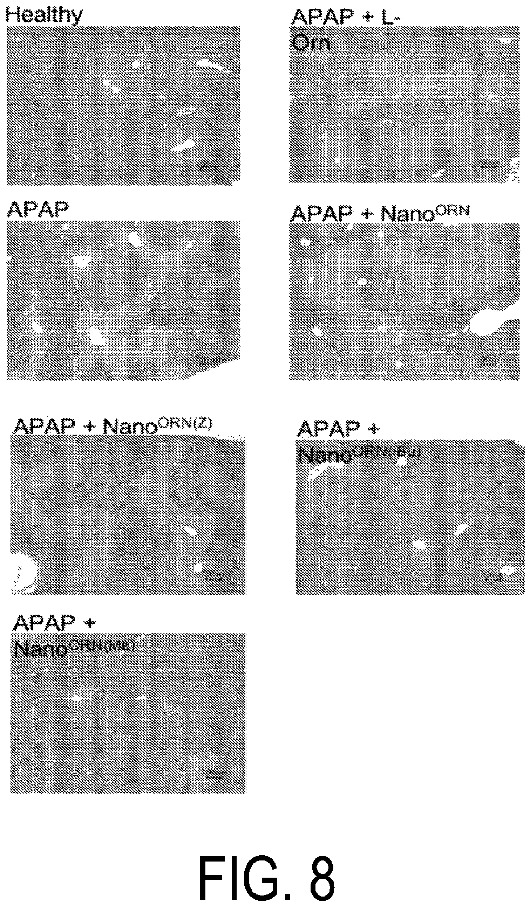

Views of hematoxylin and eosin (H & E) staining of liver tissue sections from these tests are illustrated in FIG. 8. Necrosis of liver tissue was observed in the APAP-administered group. While Nano^{ORN} and Nano^{ORN (Z)} show the same tendency, Nano^{ORN (Me)} and Nano^{ORN (iBu)} considerably suppress the necrosis.

### Test Example 3: Effect of oral administration of ornithine structure to C57/BL healthy mice(toxicity evaluation)

Six C57BL/6N mice (male) per group were forced to be orally administered with the following samples (for groups 2 to 7, the ornithine content was adjusted to about 200 mg/kg) by use of a 0.5 mL sonde. Their body weight was measured every day, and 4 days after the start of administration, whole blood was used for measurement of blood cell count, and plasma was used for measurement of hepatic disorder markers AST and ALT.
n = 6
Group 1: healthy group
Group 2: forced to be orally administered with 10 mg/mL L-ornithine (sonde)
Group 3: forced to be orally administered with 20 mg/mL PEG-b-Orn (sonde)
Group 4: forced to be orally administered with 20 mg/mL Nano^{ORN} (sonde)
Group 5: forced to be orally administered with 20 mg/mL PEG-b-POrn (Z) assembled body (Nano^{ORN (Z)}) (sonde)
Group 6: forced to be orally administered with 20 mg/mL PEG-b-POrn(COCH₃) assembled body (Nano^{ORN (Me)}) (sonde)
Group 7: forced to be orally administered with 20 mg/mL PEG-b-POrn(COCH(CH₃)₂) assembled body (Nano^{ORN (iBu)}) (sonde)

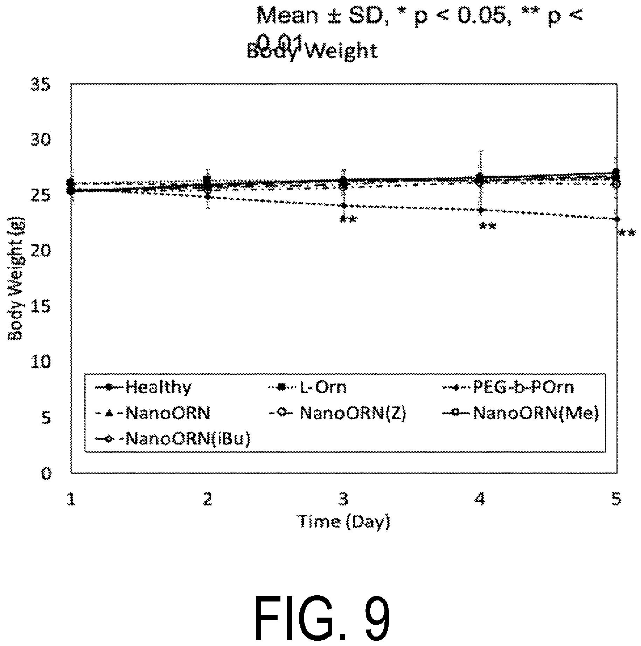

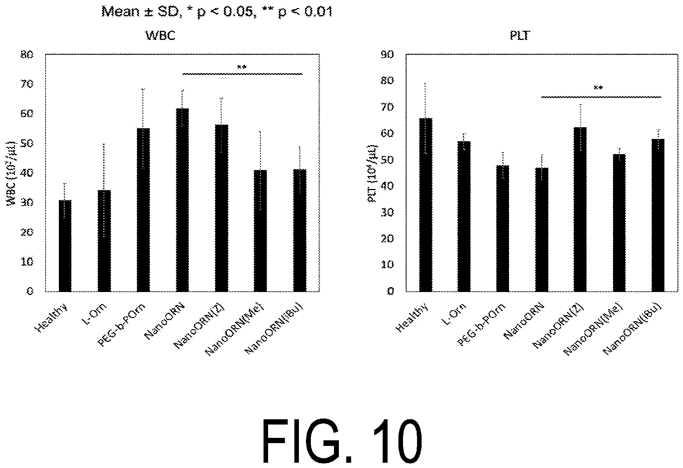

FIG. 9 shows changes in body weight of mice. Significant weight loss by virtue of forced oral administration of PEG-b-POrn is observed, confirming toxicity. In addition, as illustrated in FIG. 10, in the groups administered with PEG-b-Orn and Nano^{ORN}, an increase in leukocyte count and a decrease in platelet count are observed, but, in the case of the nano-assembled bodies, particularly Nano^{ORN(iBu)}, there is almost no change and no toxicity is exhibited.

### Test Example 4: Effect (2) of oral administration of ornithine structure to acetaminophen (APAP)-induced acute hepatic disorder mice

Six C57BL/6N mice (male) per group were forced to be orally administered with the following samples (for groups 3 to 5, the ornithine content was adjusted to about 200 mg/kg) by use of a 0.5 mL sonde. Three days after the start of administration, an aqueous APAP solution (15 mg/mL, 0.5 mL, 300 mg/Kg-BW) was intraperitoneally administered. Mouse survival was then checked for 14 days.
Group 1: healthy group
Group 2: APAP-administered group
Group 3: APAP-administered group/forced to be orally administered with 10 mg/mL L-ornithine + 10 mg/mL L-aspartic acid (sonde)
Group 4: APAP-administered group/forced to be orally administered with 20 mg/mL Nano^{ORN} (sonde)
Group 5: APAP-administered group/forced to be orally administered with 20 mg/mL PEG-b-POm(COCH(CH₃₎₂ assembled body (Nano^{ORN (iBu)}) (sonde)

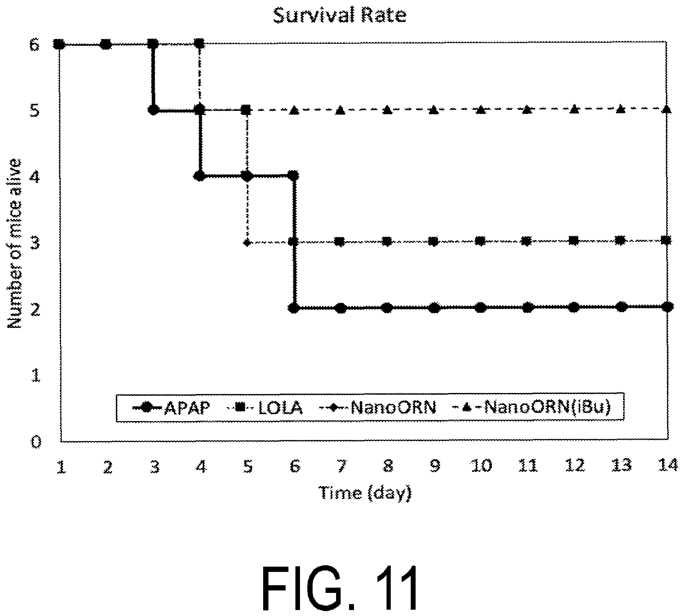

As illustrated in FIG. 11, while the survival rate was 33% after 14 days in the APAP-administered group, the survival rate was 50% in the group administered with Nano^{ORN} and 83% in the group administered with Nano^{ORN (iBu)}, which were extremely high, demonstrating a high effect.

### Reference Production Example 1: Synthesis of CH₃O-C₆H₅-CH₂O-(CH₂CH₂O)n-CH₂CH₂OSO₂CH₃ (N953 (2))

Potassium naphthalene (1 mml) and ethyleneoxide (115 mmol) were added to commercially available CH₃OCH₂CH₂OH (1 mmol) in 50 mL of ultra-dehydrated THF in a 100 mL flask, and reacted for 1 day under water cooling, and then methanesulfonylchloride (5 mmol) was added thereto. After 20 minutes, the mixture was poured into 500 mL of 2-propanol for precipitation, and then the precipitate was dissolved in methanol for precipitation. This operation was repeated twice, and the precipitate was dried under reduced pressure to give a target product

### Reference Production Example 2: Synthesis of CH₃O-C₆H₅-CH₂O-(CH₂CH₂O)n-CH₂CH₂NH₂ (N956)

The CH₃O-C₆H₅-CH₂O-(CH₂CH₂O)n-CH₂CH₂OSO₂CH₃ obtained in Experimental Example 1 was added with 600 mL of 28% aqueous ammonia, and they were reacted at 50°C for 1 day. Then, the reaction product was extracted with 50 mL of chloroform, and the chloroform phase was dehydrated with NaHSO₄ and filtered. Then, the product was precipitated in 2-propanol (IPA), and the obtained precipitate was dissolved in methanol for precipitation. This operation was repeated twice, and the precipitate was dried under reduced pressure to give a target product

### Reference Production Example 3: Synthesis of CH₃O-C₆H₅-CH₂O-(CH₂CH₂O)n-CH₂CH₂NH-(CO(CH₂CH₂CH₂NH(Z))NH)m-H (N962)

N956 obtained in Reference Production Example 2 was dissolved in DMF, and a solution of commercially available N-benzyl-aspartic acid NCA in DMF was added thereto, followed by stirring at room temperature for 2 days. The solution was added to hexane:2-propanol (8:2, 500 mL), centrifuged to give a polymer, which was dried under reduced pressure to give a target product.

### Reference Production Example 4: Synthesis of CH₃O-C₆H₅-CH₂O-(CH₂CH₂O)n-CH₂CH₂NH-(CO(CH₂CH₂CH₂NH₂)NH)m-H (PEG-b-POrn); N973)

N962 obtained in Reference Production Example 3 was dissolved in trifluoroacetic acid, ice-cooled to 0°C, and added with 3% HBr (acetic acid), and they were reacted for 4 hours. This was added to cooled 2-propanol of 800 mL to give a precipitate, which was dried under vacuum to give a target product.

### Production Example 12: CH₃O-C₆H₅-CH₂O-(CH₂CH₂O)n-CH₂CH₂NH-(CO(CH₂CH₂CH₂NHC(=O)CH(CH₃)₂)NH)m-H (PEG-b-POrn(COCH(CH₃)₂); N978)

A target polymer (1.4 g) was obtained in exactly the same manner as in Production Example 7, except that N973 synthesized in Reference Production Example 4 was used instead of N841.

### Test Example 5: Kinetic analysis by ¹²⁵I-labeled PEG-b-POrn (COCH_{(CH3})₂)

N978 synthesized in Production Example 125 was dissolved in DMSO, and the solution was dialyzed against 2 L of DI-water 2L for 72 hours (dialysis water was exchanged every 12 hours) to give an aqueous solution of an assembled body. Thereafter, chloramine T and a radioisotope ¹²⁵I of iodine were mixed, and the phenyl group introduced into a terminal of the PEG chain was labeled with ¹²⁵I.

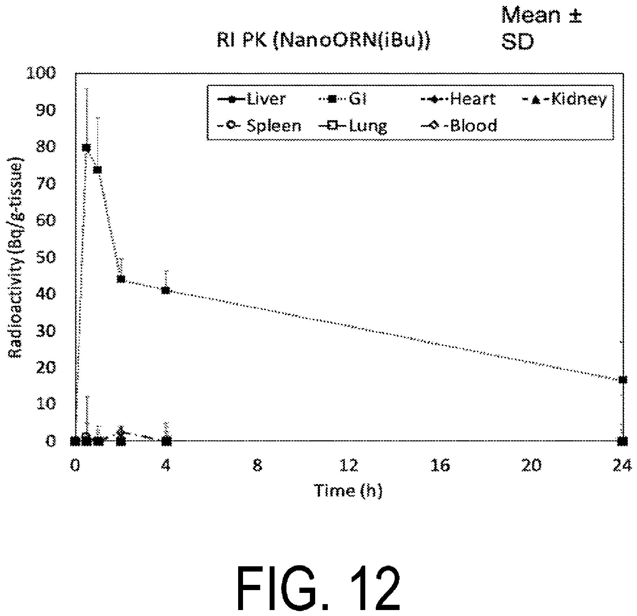

For examination of pharmacokinetics, three ICR mice were prepared for each of endpoints at 0.5 h, 1 h, 2 h, 4 h, and 24 h, i.e., 15 mice in total. After the labeled structures were forced to be orally administered by use of a sonde, the mice were sacrificed for each endpoint. Hearts, lungs, digestive tracts, livers, spleens, kidneys and bloods were extracted, and radioactivity of each organ was measured with a gamma counter. As illustrated in FIG. 12, ¹²⁵I-labeled Nano^{ORN (iBu)} was localized in the digestive tracts and was almost completely absent in bloods and organs.

### Production Example 13: Synthesis of PEG-b-Orn (iBu)-TAMRA or PEG-b-Orn (iBu)-Cy5

The former as a target product was synthesized by exactly the same method as in Production Example 7, except that 1 mg of commercially available TAMRA-N=C=S was added before isobutyric anhydride was added and that the mixture was reacted for 10 minutes.

The latter as a target product was synthesized by exactly the same method as in Production Example 7, except that 1 mg of commercially available Cy5-NHS was added before isobutyric anhydride was added and that the mixture was reacted for 10 minutes.

### Test Example 6: In vitro toxicity evaluation of Nano^{Porn}

BAEC (bovine aortic endothelial cells), L-929 (mouse fibroblasts) and RAW264.7 (mouse macrophages) were prepared, and seeded, at from 5 to 10 × 10³ cells, in 96-well plates. Various concentrations of Nano^{ORN (Z)}, Nano^{ORN (Me)}, Nano^{ORN (iBu)}, and Nano^{ORN} were added to each well. After 24 or 48 hours of culture, MTT reagents were added. After additional 4 hours, DMSO was added, and formazan crystals were solubilized. The absorption at a wavelength of 575 nm was measured.

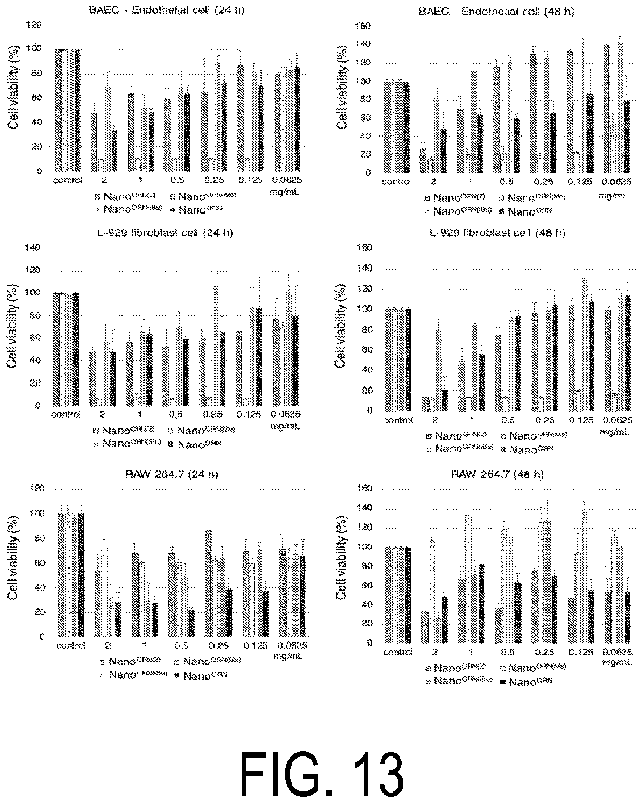

The results are presented in FIG. 13. Nano^{ORN (Z)} and Nano^{ORN (Me)} were relatively highly toxic to L-929 cells, and Nano^{ORN} was relatively highly toxic to RAW264.7 macrophage cells, whereas Nano^{ORN (iBu)} was hardly cytotoxic and was very safe.

### Production Example 14: Synthesis of CH₃O-(CH₂CH₂O)n-CH₂H₂NH-(COCH(CH₂COOCH₂Ph)NH)m-H (N865) (PEG-b-PBLA)

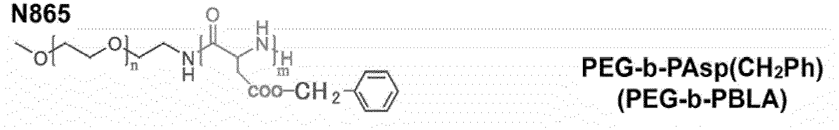

N819 (10 g) synthesized according to Production Example 2 was dissolved in 60 mL of DMF. Commercially available L-aspartic acid (CH₂Ph)-N-carboxylic anhydride (L-ASP (Z)-NCA) (10 g) was dissolved in 40 mL of DMF. They were mixed and reacted at room temperature for 2 days. The resulting polymer was precipitated in 2-propanol (IPA), then the precipitate was dissolved in acetone and precipitated in hexane. This operation was repeated twice, and the precipitate was dried under reduced pressure to give a target product (yield: 16 g; n = 114; m = 15.5)

### Production Example 15: Synthesis of L-aspartic acid (Me)-N-carboxylic anhydride (L-ASP (Me)-NCA) (N871)

Commercially available methyl L-aspartate -hydrochloride (15 g; 81.6 mmol) was dissolved in 90 mL of THF, and α-pinene (33 g; 252 mmol) and triphosgene 12.2 g (40.8 mmol) were added, and they were reacted at 50°C for 2 hours. The reaction solution is poured into 1 L of hexane, and the precipitate is filtered, dissolved in 100 mL of 2-propanol, precipitated in 1 L of hexane, and filtered. This operation was repeated twice, and the resulting precipitate was dried under reduced pressure to give a target product (yield: 10.2 g).

### Production Example 16: Synthesis of CH₃O-(CH₂CH₂O)n-CH₂H₂NH-(COCH(CH₂COOCH₃)NH)m-H (N869) (PEG-b-PASP (Me))

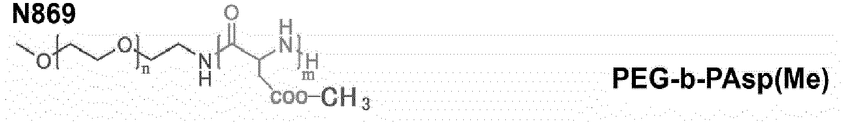

N819 (5 g) synthesized according to Production Example 2 was dissolved in 30 mL of DMF. L-aspartic acid (CH₃)-N-carboxylic anhydride (L-ASP (Me)-NCA) (5 g) synthesized in Production Example 15 was dissolved in 20 mL of DMF. They were mixed and reacted at room temperature for 2 days. The resulting polymer was precipitated in 2-propanol (IPA), then dissolved in methanol, and precipitated in ether. This operation was repeated twice, and the precipitate was dried under reduced pressure to give a target product (yield: 6.4 g; n = 114; m = 10)

### Production Example 17: Synthesis of PEG-b-P(Orn (Z)-co-Asp (CH₂Ph)) (N877)

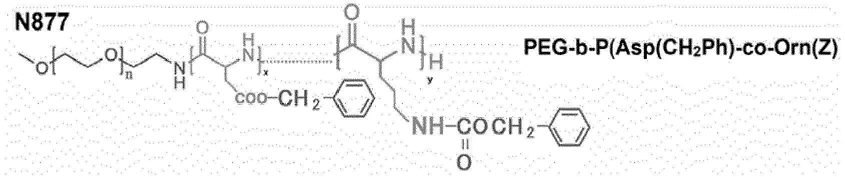

PEG-NH₂ (N866) (5 g; 1 mmol) synthesized according to Production Example 2 was dissolved in 30 mL of DMF.

L-Orn (Z)-NCA (N854) (6 g; 20 mmol) synthesized according to the Production Example and commercially available L-aspartic acid (CH₂Ph)-N-carboxylic anhydride (L-ASP (Z)-NCA) (2.5 g; 10 mmol) were dissolved in 20 mL of DMF. They were mixed and reacted at room temperature for 2 days. The resulting polymer was precipitated in 2-propanol (IPA)/hexane (1:9 v), then the precipitate was dissolved in acetone and precipitated in hexane. This operation was repeated twice, and the precipitate was dried under reduced pressure to give a target product (yield: 8.8 g; n = 114; number of asparagine units = 15; number of ornithine units = 20)

### Production Example 18: Preparation of PEG-b-PBLA assembled body (Nano^{ASP (Bz)})

N865 (1 g) synthesized according to Production Example 14 was dissolved in 20 mL of DMF, 20 mL of DI-water was added, the solution was placed in a dialysis membrane (molecular weight cut off (MWCO) = 12KDa - 14KDa), and dialyzed for 72 hours against 2 L of DI-water 2L (dialysis water was exchanged every 12 hours) to give an aqueous solution containing a target assembled body.

### Production Example 19: Preparation of PEG-b-PASP (Me) assembled body (Nano^{ASP (Me}))

The same procedures as in Production Example 18 were repeated, except that N869 (1 g) synthesized according to Production Example 16 was used, to give an aqueous solution containing a target assembled body.

Production Example 20: Preparation of PEG-b-P(Orn (Z)-co-Asp (CH₂Ph)) structure (Nano^{ASP/ORN}) (N978)

The same procedures as in Production Example 18 were repeated, except that N877 (1 g) synthesized according to Production Example 17 was used, to give an aqueous solution containing a target assembled body.

### Test Example 7: Stability evaluation of Nano^{ASP (Bz)}, Nano^{ASP (Me)}, and Nano^{ASP/ORN}

The particle diameters of the particles prepared in Production Examples 16 to 18 were measured by a dynamic light scattering method.

Nano^{ASP (Bz)} formed stable nanoparticles of about from 120 to 140 nm at a pH of up to 12 over 24 hours.

Nano^{ASP (Me)} forms an assembled body of about 10 nm for up to 6 hours at all measurement pHs. After 24 hours, aggregates of about 100 nm were observed at pH 1 and 5.

Nano^{ASP/ORN} formed an assembled body of about 100 nm for up to 24 hours at all measurement pHs.

In addition, Nano^{ASP (Bz)}, Nano^{ASP (Me)}, and Nano^{ASP/ORN} did not show substantial toxicity to mice similarly to the PEG-b-POrn (Z) assembled body (Nano^{ORN (Z)}) and the PEG-b-POrn (Me) assembled body (Nano^{ORN (Me)}) according to the results of changes in body weight of the orally administered (sonde) acute hepatic disorder mice.

### Test Example 7 Effect of oral administration of samples for the following groups to acetaminophen (APAP)-induced acute hepatic disorder mice

The method of Test Example 2 was repeated except that samples for the following groups were used.
Group 1: healthy group
Group 2: APAP-administered group
Group 3: APAP-administered group/orally administered with 200 mg/kg L-aspartic acid (sonde)
Group 4: APAP-administered group/orally administered with 200 mg/kg PEG-b-POrn (Z) assembled body (Nano^{ORN (Z)}) (sonde)
Group 5: APAP-administered group/orally administered with 200 mg/kg PEG-b-POrn (Me) assembled body (Nano^{ORN (Me)}) (sonde)
Group 6: APAP-administered group/orally administered with 200 mg/kg PEG-b-PBLA assembled body (Nano^{ASP (Me)}) (sonde)
Group 7: APAP-administered group/orally administered with 200 mg/kg PEG-b-PAsp (Me) assembled body (Nano^{ORN (Bz))} (sonde)
Group 8: APAP-administered group/orally administered with 200 mg/kg PEG-b-PAsp (Me) assembled body (Nano^{ASP/ORN)} (sonde)

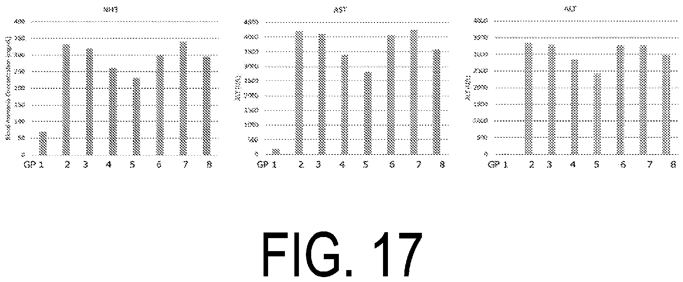

As a result of the above test, these assembled bodies provide a weight change equivalent to that of the healthy group, were hardly toxic, and provide blood ammonia levels, AST and ALT levels of the acute hepatic disorder mice as illustrated in FIG. 17.

### Production Example 21: Synthesis of PEG-b-PArg(Ac)₂ (N1047)

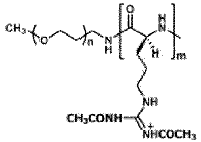

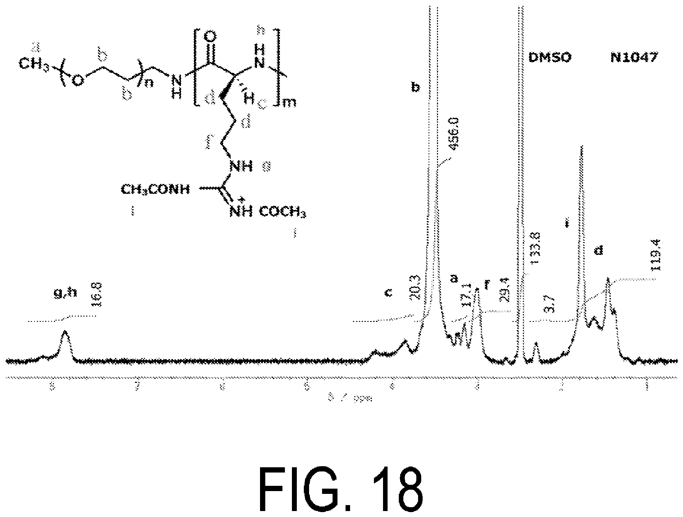

PEG-b-PArg (N1046; 2.4 g) synthesized in the same manner as in Example 5 of PCT/JP2016/62062 A1 was dissolved in 50 mL of DMF. Acetic anhydride (30 mL) was added to this, and there were reacted at 50°C for 5 hours and then at room temperature for 1 day. The resulting polymer is precipitated in t-butyl methyl ether and then washed with hexane. This operation was repeated twice, and the precipitate was dried under reduced pressure to give a target product (yield: 2.4 g; n = 114; m = 28). A ¹H-NMR chart of the target copolymer is illustrated in FIG. 18.

### Production Example 22: Synthesis of PEG-b-PArg (iBu)₂ (N1063)

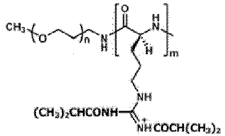

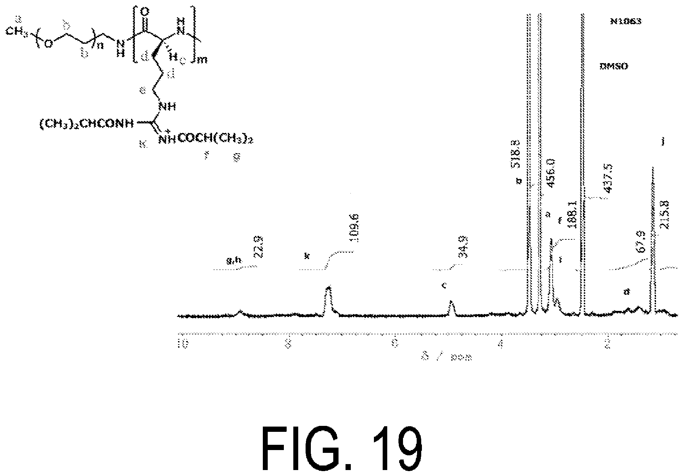

A target product was obtained in exactly the same manner as in Production Example 21 except that acetic anhydride was replaced with isobutyric anhydride (yield: 2.4 g; n = 114; m = 28). A ¹H-NMR chart of the target copolymer is illustrated in FIG. 19.

### Production Example 23: Preparation of assembled body of PEG-b-PArg (Ac)₂ (Nano^{Arg (Ac)})

N1047 (1 g) synthesized according to Production Example 21 was dissolved in 20 mL of DMF, 20 mL of DI-water was added, the solution was placed in a dialysis membrane (molecular weight cut off (MWCO) = 12KDa - 14KDa), and dialyzed for 72 hours against 2 L of DI-water 2L (dialysis water was exchanged every 12 hours) to give an aqueous solution containing Nano^{Arg (Ac)}. (Average particle diameter: 132 nm, ζ potential: + 4.9 mV)

### Production Example 24: Preparation of assembled body of PEG-b-PARG (iBu)₂ (Nano^{Arg (iBu)})

An assembled body was prepared in exactly the same manner as in Production Example 23 except that the N1063 (1 g) synthesized in Production Example 22 was used. Average particle diameter: 44 nm, ζ potential: -2.2 mV)

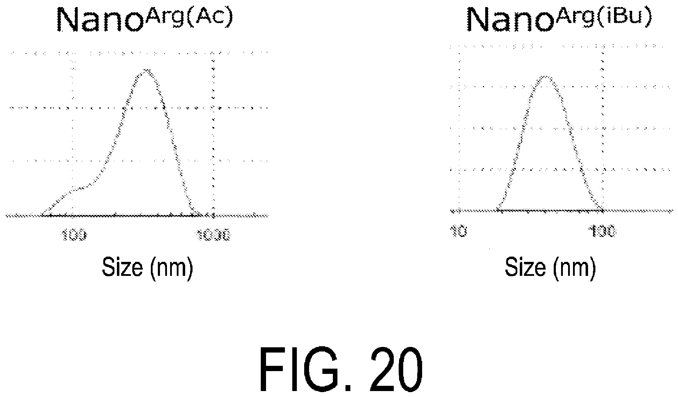

FIG. 20 illustrates measurement results of light scattering particle diameters in aqueous solutions of Nano^{Arg (Ac)} and Nano^{Arg (iBu)}.

### Test Example 8: Effect of oral administration and intravenous injection of acetylated arginine assembled body to acetaminophen (APAP)-induced acute hepatic disorder mice

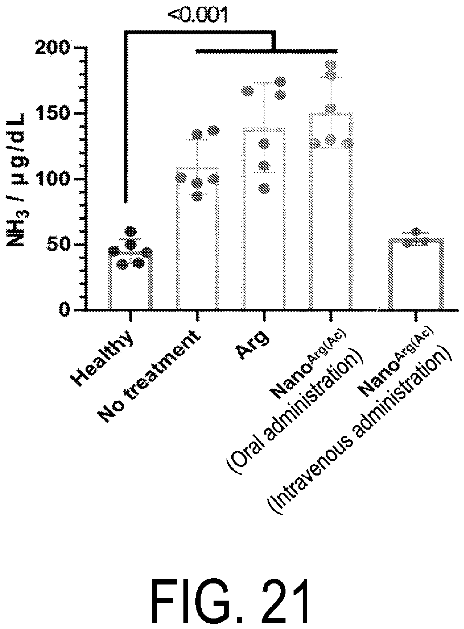

The procedure of Test Example 2 was repeated except that the acetylated arginine assembled body was used in place of the ornithine assembled body of Test Example 2. The results are shown in FIG. 21. As illustrated in FIG. 21, the acetylated polyarginine particles (Nano^{Arg (Ac)}) showed no effect when administered orally, but significantly reduced the blood ammonia concentration when administered intravenously.

### Test Example 9: Effect of oral administration of acetylated arginine structure to nonalcoholic steatohepatitis (NASH) model mice

Six C57BL/6J mice (male) per group were allowed to be freely orally administered with an aqueous solution of acetylated polyarginine particles (Nano^{Arg (Ac)} ([Nano^{Arg (Ac)}] = 5 mg/mL) (Days 0 to 8). One day after the start of oral administration, a choline-deficient methionine-reduced high-fat diet (CADHFD) was given (Days 1 to 8). Bloods and livers were collected on Day 8 after the start of CADHFD feeding. Plasma was used for measurement of triglyceride (TG).
GP1: healthy group
GP2 CADHFD-fed group
GP3 CADHFD-fed group/orally administered with (allowed to freely drink) 5 mg/mL acetylated polyarginine particle (Nano^{Arg (Ac)}) aqueous solution
GP4 CADHFD-fed group/orally administered with (allowed to freely drink) 1.5 mg/mL L-arginine aqueous solution

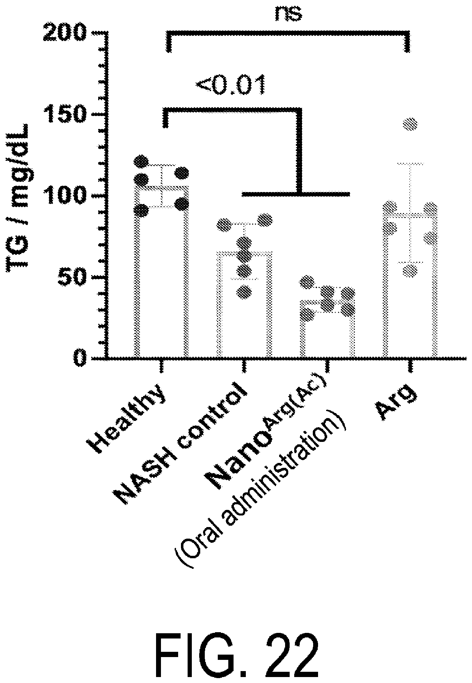

The results are illustrated in FIG. 22. It can be seen from the figure that triglycerides are significantly reduced by oral administration.

### Industrial Applicability

The copolymer and the ornithine microparticles according to the present invention can be used as, for example, medicaments for the prevention and treatment of hepatic dysfunction, though not limited thereto, and can be utilized at least in the pharmaceutical industry.

## Claims

1. A copolymer represented by Formula (I): 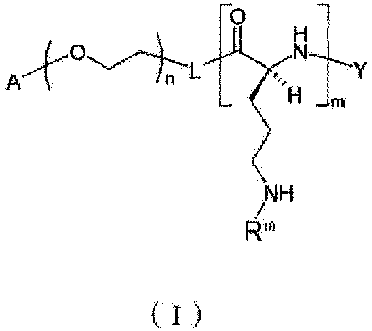 wherein
A represents:
(i) a hydrogen atom, an unsubstituted or substituted C₁-C₁₂ alkyl group, an unsubstituted or substituted C₁-C₁₂ alkoxy group, or an unsubstituted or substituted aryl group,
when the A is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-; or
(ii) formula 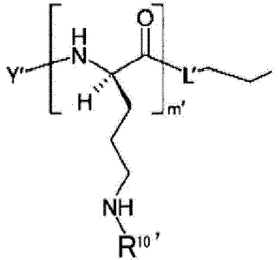 where
L and L' independently represent a linking group;
Y and Y' independently represent a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
R¹⁰ and R¹⁰' are hydrogen atoms or (R¹¹-(C=O)-)'s, where (R¹¹)'s are each independently substituted or unsubstituted C₁-C₂₁ alkyl, substituted or unsubstituted aryl, or substituted or unsubstituted C₁-C₂₁ alkoxy,
when the R¹¹ is substituted, substituents are C₁₋₄ alkyl, C₁₋₄ alkoxy, or aryl;
m and m' are independently an integer of from 2 to 300;
n is an integer of from 2 to 1,000; and
20% or more of m or m' (R¹⁰)'s and (R¹⁰')'s are R¹¹-(C=O)-.

2. The copolymer according to claim 1, wherein R¹⁰ in formula (I) is R¹¹-(C=O)-, and 100% of m (R¹⁰)'s are (R¹1-(C=O)-)'s.

3. The copolymer according to claim 1, wherein A is defined as (i).

4. The copolymer according to claim 1, wherein A is defined as (i), R¹⁰ is R¹¹-(C=O)-, and 100% of m (R¹⁰)'s are (R¹¹-(C=O)-)'s.

5. The copolymer according to claim 1, wherein A is defined as (ii).

6. The copolymer according to claim 1, wherein A is defined as (ii), R¹⁰ and R¹⁰' are (R¹¹-(C=O)-)'s, and 100% of m and m' (R¹⁰)'s and (R¹⁰')'s are (R¹¹-(C=O)-)'s.

7. An ornithine microparticle comprising the copolymer described in any one of claims 1 to 6, wherein the ornithine microparticle has an average particle size of from 1 nanometer to 100 micrometers.

8. A pharmaceutical composition comprising the copolymer described in any one of claims 1 to 6 as an active ingredient and an additive.

9. The pharmaceutical composition according to claim 8, wherein the pharmaceutical composition is an orally administered agent.

10. A composition for preventing or treating hepatic dysfunction, the composition comprising the copolymer described in any one of claims 1 to 6 or the ornithine microparticle described in claim 7 as an active ingredient.

11. A pharmaceutical composition comprising the copolymer described in any one of claims 1 to 6 or the ornithine microparticle described in claim 7 as an active ingredient and a copolymer represented by formula (IV): 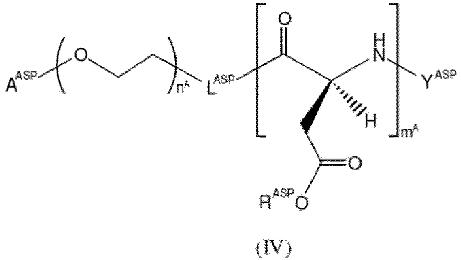 wherein
A^{ASP} represents a hydrogen atom, an unsubstituted or substituted C₁-C₁₂ alkyl group, an unsubstituted or substituted C₁-C₁₂ alkoxy group, or an unsubstituted or substituted aryl group,
when the A^{ASP} is substituted, a substituent represents a C₁-C₄ alkyl group, a C₁-C₄ alkoxy group, an aryl group, a formyl group, or a group of formula R₁R₂CH- (wherein R¹ and R² independently represent C₁-C₄ alkoxy, or R₁ and R₂ together represent -OCH₂CH₂O-, - O(CH₂)₃O-, or -O(CH₂)₄O-);
L^{ASP} represents a linking group;
R^{ASP} is a hydrogen atom, substituted or unsubstituted C₁-C₂₁ alkyl, or substituted or unsubstituted aryl,
when the substituent is substituted, a substituent is C₁₋₄ alkyl, C₁₋₄ alkoxy, or aryl;
Y^{ASP} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residues,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
m^{A} is an integer of from 2 to 300; and
n^{A} is an integer of from 2 to 1,000.

12. A pharmaceutical composition comprising the copolymer described in any one of claims 1 to 6 or the ornithine microparticle described in claim 7 as an active ingredient and a copolymer represented by formula (V): 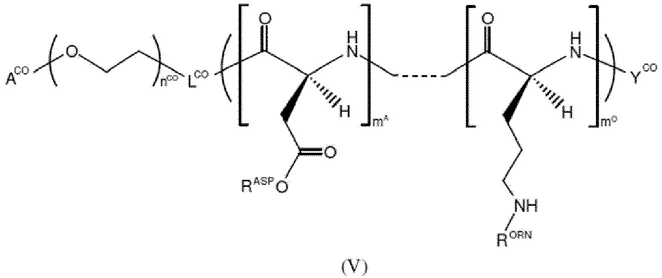 wherein
A^{CO} represents a hydrogen atom, an unsubstituted or substituted C₁-C₁₂ alkyl group, an unsubstituted or substituted C₁-C₁₂ alkoxy group, or an unsubstituted or substituted aryl group,
when the A^{CO} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
n^{CO} is an integer of from 2 to 1,000;
L^{CO} represents a linking group;
R^{ASP} is a hydrogen atom, unsubstituted or substituted C₁-C₂₁ alkyl, or unsubstituted or substitute aryl,
when the R^{ASP} is substituted, a substituent is C₁₋₄ alkyl, C₁₋₄ alkoxy, or aryl;
m^{A} is an integer of from 2 to 300,
R^{ORN} is a hydrogen atom or R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
when the R¹¹ is substituted, a substituent is C₁₋₄ alkyl, C₁₋₄ alkoxy, or aryl;
m^{O} is an integer of from 2 to 300; and
Y^{CO} represents a hydrogen atom, C₁₋₂₁ alkylcarbonyl, unsubstituted or substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl;
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino,
where amino acids in m^{A} repeat units and m^{O} repeat units are each randomly present and form another block.

13. A pharmaceutical composition comprising the copolymer described in any one of claims 1 to 6 or the ornithine microparticle described in claim 7 as an active ingredient and a copolymer represented by formula (VI): 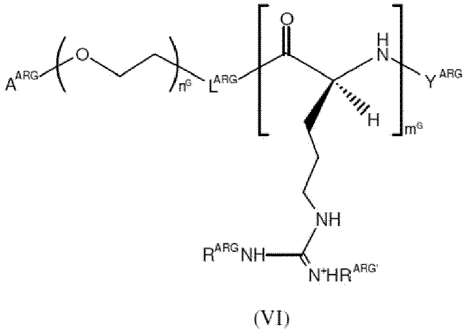 wherein
A^{ARG} represents a hydrogen atom, an unsubstituted or substituted C₁-C₂₁ alkylcarbonyl, unsubstituted or substituted arylcarbonyl, or unsubstituted or substituted C₁-C₂₁ alkoxycarbonyl,
when the A^{ARG} is substituted, a substituent is C₁₋₄ alkyl, C₁₋₄ alkoxy, or aryl;
n^{G} is an integer of from 2 to 1,000;
L^{ARG} represents a linking group;
R^{ARG} and R^{ARG'} are independently a hydrogen atom or R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
when the R¹¹ is substituted, a substituent is C₁₋₄ alkyl, C₁₋₄ alkoxy, or aryl;
m^{G} is an integer of from 2 to 300; and
Y^{ARG} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residues,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino.

14. A copolymer represented by Formula (IV-a): 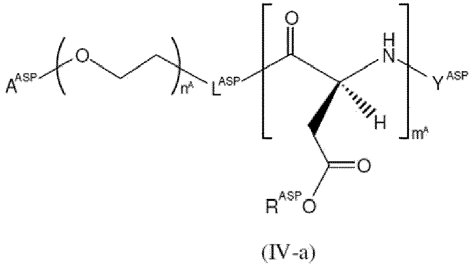 wherein
A^{ASP} represents a hydrogen atom, an unsubstituted or substituted C₁-C₁₂ alkyl group, an unsubstituted or substituted C₁-C₁₂ alkoxy group, or an unsubstituted or substituted aryl group,
when the A^{ASP} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
L^{ASP} represents a linking group;
Y^{ASP} represents C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residues,
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino;
R^{ASP} is substituted or unsubstituted C₁-C₂₁ alkyl, or substituted or unsubstituted aryl,
when the R^{ASP} is substituted, a substituent is C₁₋₄ alkyl, C₁₋₄ alkoxy, or aryl, provided that they are other than benzyl;
m^{A} is an integer of from 2 to 300; and
n^{A} is an integer of from 2 to 1,000.

15. A copolymer represented by Formula (V): 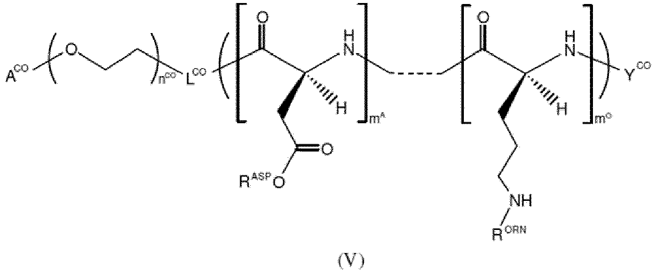 wherein
A^{CO} represents a hydrogen atom, an unsubstituted or substituted C₁-C₁₂ alkyl group, an unsubstituted or substituted C₁-C₁₂ alkoxy group, or an unsubstituted or substituted aryl group,
when the A^{CO} is substituted, a substituent represents a C₁-C₄ alkyl group, a C₁-C₄ alkoxy group, an aryl group, a formyl group, or a group of formula R¹R²CH- (wherein R¹ and R² independently represent C₁-C₄ alkoxy, or R¹ and R² together represent -OCH₂CH₂O-, - O(CH₂)₃O-, or -O(CH₂)₄O-);
n^{CO} is an integer of from 2 to 1,000;
L^{CO} represents a linking group;
R^{ASP} is a hydrogen atom, substituted or unsubstituted C₁₋₂₁ alkyl, or substituted or unsubstituted aryl,
when the R^{ASP} is substituted, a substituent is C₁₋₄ alkyl, C₁₋₄ alkoxy, or aryl;
m^{A} is an integer of from 2 to 300,
R^{ORN} is a hydrogen atom or R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
when the R¹¹ is substituted, a substituent is C₁₋₄ alkyl, C₁₋₄ alkoxy, or aryl;
m^{O} is an integer of from 2 to 300; and
Y^{CO} represents a hydrogen atom, C₁₋₂₁ alkylcarbonyl, unsubstituted or substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl;
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue,
when the substituent is substituted, a substituent may be C₁₋₄ alkyl, C₁₋₄ alkyloxy, hydroxyl, carboxyl, cyano, nitro, halogen atoms, or mono- or di-C¹⁻⁴ alkylamino,
where amino acids in m^{A} repeat units and m^{O} repeat units are each randomly present and form another block.

16. A copolymer represented by formula (VI-a): 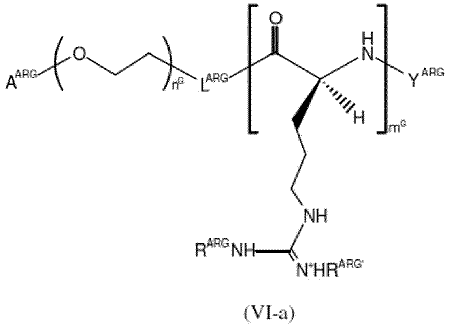 wherein
A^{ARG} represents a hydrogen atom, a substituted or unsubstituted C₁-C₂₁ alkylcarbonyl group, substituted or unsubstituted arylcarbonyl, or substituted or unsubstituted C₁-C₂₁ alkoxycarbonyl,
when the A^{ARG} is substituted, a substituent is C₁₋₄ alkyl, C₁₋₄ alkoxy, or aryl;
n^{G} is an integer of from 2 to 1,000;
L^{ARG} represents a linking group;
R^{ARG} and R^{ARG'} are independently R¹¹-(C=O)-, wherein (R¹¹)'s are each independently substituted or unsubstituted C₁-C₂₁ alkyl, substituted or unsubstituted aryl, or substituted or unsubstituted C₁-C₂₁ alkoxy,
when the R¹¹ is substituted, a substituent is C₁₋₄ alkyl, C₁₋₄ alkoxy, or aryl, provided that they are other than benzyloxycarbonyl and tert-butyloxycarbonyl;
m^{G} is an integer of from 2 to 300; and
Y^{ARG} represents a hydrogen atom, C₁₋₂₁ alkylcarbonyl, substituted C₁₋₄ alkylcarbonyl, unsubstituted or substituted C₃₋₇ cycloalkylcarbonyl, unsubstituted or substituted arylcarbonyl, or unsubstituted or substituted 5- or 6-membered heteroarylcarbonyl,
where a substituent of the substituted C₁₋₄ alkylcarbonyl is selected from the group consisting of a halogen atom, hydroxyl, carboxyl, unsubstituted or substituted C₃₋₇ cycloalkyl, unsubstituted or substituted aryl, and unsubstituted or substituted 5- or 6-membered heteroaryl, unsubstituted or substituted adamantyl, and unsubstituted or substituted cholesterol residue, and
when the substituent is substituted, a substituent may be C₁₋₄ alkyl, C₁₋₄ alkyloxy, hydroxyl, carboxyl, cyano, nitro, halogen atom, or mono- or di-C₁₋₄ alkylamino.
